# FASCICULE DE BREVET EUROPEEN

(11) **EP 3 756 123 B1**
(45) Date de publication et mention de la délivrance du brevet: **06.04.2022**
(21) Numéro de dépôt: 19705362.2
(22) Date de dépôt: 19.02.2019
(51) Int. Cl.: G16H 10/60, G06F 21/62, G16H 30/20

(54) **PROCEDE DE RE-IDENTIFICATION D'UN OBJET D'INFORMATIONS MEDICALES, PROCEDE DE DE-IDENTIFICATION D'UN OBJET D'INFORMATIONS MEDICALES, DISPOSITIFS, EQUIPEMENT DE COMMUNICATION ET PROGRAMMES D'ORDINATEUR ASSOCIES**
VERFAHREN ZUR ERNEUTEN IDENTIFIZIERUNG EINES OBJEKTS VON MEDIZINISCHER INFORMATION, VERFAHREN ZUR ENTIDENTIFIZIERUNG EINES OBJEKTS VON MEDIZINISCHER INFORMATION, VORRICHTUNGEN, KOMMUNIKATIONSAUSRÜSTUNGSARTIKEL UND DAMIT ASSOZIIERTE COMPUTERPROGRAMME
METHOD OF RE-IDENTIFICATION OF A MEDICAL-INFORMATION OBJECT, METHOD OF DE-IDENTIFICATION OF A MEDICAL-INFORMATION OBJECT, DEVICES, COMMUNICATION EQUIPMENT ITEM AND COMPUTER PROGRAMS ASSOCIATED THEREWITH

(30) Priorité: 23.02.2018 FR 1851629
(43) Date de publication de la demande: 30.12.2020
(73) Titulaire: Fondation B Com, 35510 Cesson Sevigne (FR)
(72) Inventeur: PASQUIER, Guillaume, 35700 RENNES (FR); LE MAITRE, Amandine, Redondo Beach, California 90278 (US); CORDONNIER, Emmanuel, 35000 RENNES (FR)
(74) Mandataire: Ermeneux, Bertrand
(86) Numéro de dépôt international: PCT/EP2019/054063
(87) Numéro de publication internationale: WO 2019/162258

(56) Documents cités:
- US-A1- 2011 110 568
- US-A1- 2016 154 977
- "Digital Imaging and Communications in Medicine (DICOM) 8 Supplement 142: Clinical Trial De-identification Profiles DICOM Standards Committee, Working Group 18 Clinical Trials Table of Contents", , 25 janvier 2011 (2011-01-25), pages 1-44, XP055182476, Rosslyn, VA, USA Extrait de l'Internet: URL:ftp://medical.nema.org/medical/dicom/f inal/sup142_ft.pdf [extrait le 2015-04-13] cité dans la demande
- CHUNG-YUEH LIEN ET AL: "Open source tools for standardized privacy protection of medical images", PROCEEDINGS OF SPIE, vol. 7967, 3 mars 2011 (2011-03-03), page 79670M, XP055532582, 1000 20th St. Bellingham WA 98225-6705 USA ISSN: 0277-786X, DOI: 10.1117/12.877989 ISBN: 978-1-5106-2099-5
- Dicom Standards Committee: "Digital Imaging and Communications in Medicine (DICOM) Supplement 59: Key Object Selection Document SOP Class", , 25 mai 2001 (2001-05-25), pages 1-19, XP055532040, Rosslyn, Virginia 22209 USA Extrait de l'Internet: URL:ftp://medical.nema.org/medical/dicom/f inal/sup59_ft.pdf [extrait le 2018-12-10]

## Description

### 1. Domaine de l'invention

Le domaine de l'invention est celui du transfert de données médicales, telles que des images, entre un site clinique et un site non clinique.

Plus précisément l'invention concerne l'identification par le site clinique d'une image résultant d'un traitement réalisé au sein du site non clinique sur une image d'origine anonymisée et reçue dans un objet conteneur d'informations médicales.

L'invention peut notamment, mais non exclusivement, s'appliquer à des objets conteneurs d'informations médicales, dont le format est spécifié par une norme internationale, comme par exemple le standard DICOM (pour « Digital Imaging and Communications in Medicine », en anglais).

### 2. Présentation de l'art antérieur

Le standard DICOM, reconnu comme norme internationale par l'ISO (pour « International Standard Organisation », en anglais), permet d'échanger des images sous forme numérique, en maintenant en permanence un lien entre ces images elles-mêmes et des informations associées (nom du patient, type d'examen, nom de l'organe visible sur l'image, etc.), appelées métadonnées.

Cette norme décrit un objet d'informations médicales, ou conteneur, comprenant notamment :
- la ou les images;
- des métadonnées associées à ces images, comprenant :
   ∘ des métadonnées non identifiantes, telles que par exemple les dimensions de l'image ;
   ∘ des métadonnées dites identifiantes, comme par exemple un identifiant de l'image dans l'objet. Ces métadonnées identifiantes comprennent des métadonnées de rattachement de l'objet d'informations médicales à des données patient, telle que le nom du patient ou sa date de naissance, ou encore un identifiant d'étude ou enfin un identifiant de série ou de séquence d'images, auxquelles appartient l'image.

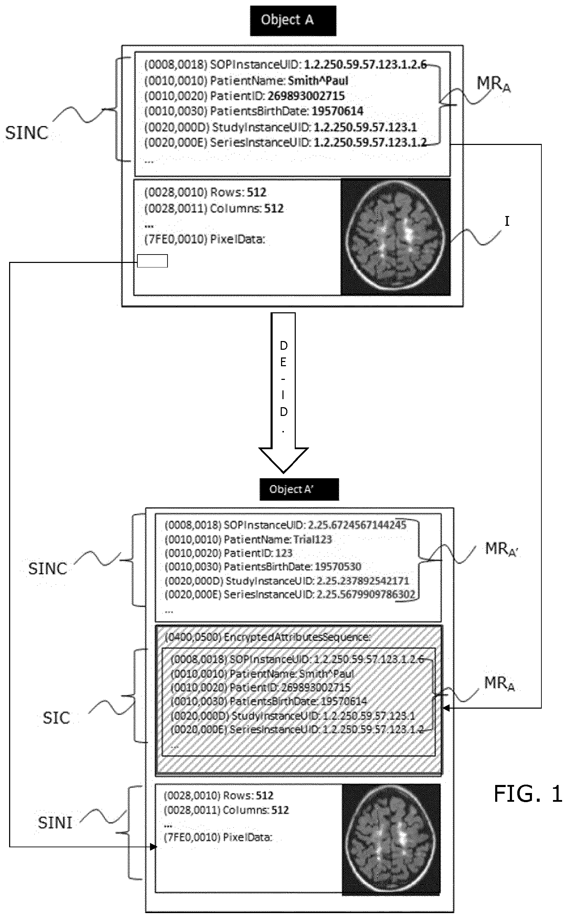

En relation avec la Figure **1****,** on a représenté un exemple d'objet A, dit principal, comprenant dans un champ d'information intitulé « données pixel » (pour « PixelData », en anglais), une image I de cerveau acquise par un équipement d'Imagerie par Résonance Magnétique (IRM). Cette image est de taille 512x512 comme indiqué dans les métadonnées non identifiantes contenues dans les champs intitulés lignes (pour « Rows », en anglais) et colonnes (pour « Columns », en anglais). Ces champs appartiennent à une séquence SINI_{A} de champs d'informations non identifiantes.

L'objet A comprend un identifiant ID_{A} de cette image contenu dans le champ intitulé « SOPInstanceUID » et aussi des métadonnées de rattachement MR_{A}, telles que le nom du patient « Paul Smith », contenu dans le champ intitulé « PatientName » ou l'identifiant de ce patient « 269893002715 » contenu dans le champ « PatientID ».

L'image appartient à une séquence ou série d'images, qui fait elle-même partie d'une étude ou examen. On notera que les métadonnées identifiantes ID_{A} + MR_{A} telles que l'identifiant d'étude (pour « StudyInstanceUID » en anglais) qui stocke la valeur « 1.2.250.59.57.123.1 » , l'identifiant de série dans l'étude (pour « SeriesInstanceUID », en anglais) qui stocke la valeur « 1.2.250.59.57.123.1.2 » ou encore le champ correspondant au numéro d'image ID_{A} dans la série (pour « SOPInstanceUID », en anglais) qui stocke la valeur « 1.2.250.59.57.123.1.2.6 », n'identifient pas directement le patient, mais permettent de remonter jusqu'à lui. Ce sont donc des métadonnées de nature à l'identifier.

Au sein du site clinique, l'accès à l'objet principal A est sécurisé et les valeurs des métadonnées identifiantes sont stockées en clair dans l'objet conteneur A, dans une séquence de champs d'informations non chiffrées SINC_{A}.

En revanche, avant tout transfert en dehors du site clinique, par exemple vers un environnement de recherche, l'objet A doit être dé-identifié.

L'opération de dé-identification, connue de l'homme de métier et par exemple spécifiée dans la section « E.3.10 Retain Safe Private Option » de la norme DICOM « PS 3.15 Security and System Management Profiles », consiste à créer un objet d'informations médicales A', dit dé-identifié, comprenant son propre identifiant d'image ID_{A'} dans le champ intitulé « SOPInstanceUID ». On recopie les informations contenues dans les champs non identifiants de l'objet A, comme l'image I et ses dimensions dans la séquence d'informations non identifiantes SINI_{A'}. En ce qui concerne les métadonnées identifiantes ID_{A} et MR_{A} de l'objet A, elles sont chiffrées puis stockées dans un champ d'information dédié de l'objet A'.

Dans l'exemple de la Figure **1****,** il s'agit d'une séquence de champs de chiffrées SIC_{A'} (pour « EncryptedAttributesSequence », en anglais) de l'objet A' dans lequel on stocke les métadonnées chiffrées relatives à toutes les métadonnées identifiantes et de rattachement de l'objet A, telles que le nom, la date de naissance du patient ou le numéro d'étude, de série ou d'image.

Les champs de la séquence SINC_{A'} correspondant à ces métadonnées identifiantes sont remplis dans l'objet A' par des valeurs factices MR_{A'}. En relation avec la Figure **1****,** on note par exemple que la valeur factice « 2.25.6724567144245 » a été écrite dans le champ « Numéro d'image » et que la valeur factice « Trial123 » remplace le véritable nom du patient dans le champ « Nom du Patient ».

Une fois cette opération de dé-identification de l'objet A effectuée, l'objet dé-identifié A' peut être transmis via un réseau de communication à un site non clinique, tel que par exemple un environnement de recherche, sans risque de divulgation des informations personnelles du patient.

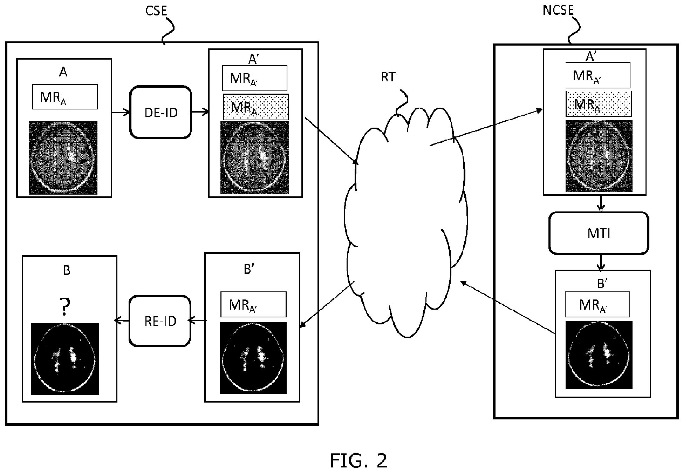

En relation avec la Figure **2****,** on a représenté un équipement de communication CSE d'un site clinique apte à communiquer avec un équipement de communication NCSE d'un site non clinique, par exemple un site de recherche, par l'intermédiaire d'un réseau de télécommunications RT, par exemple Internet, et d'une connexion sécurisée par un protocole de transfert sécurisé tel que le protocole HTTPS (pour « Hypertext Transfer Protocol Secure », en anglais). L'équipement CSE dé-identifie l'objet A comprenant l'image I en un objet A', qu'il transmet à l'équipement NCSE. Selon la norme DICOM par exemple, l'objet A' peut être échangé entre les deux équipements, soit en mode « push » en utilisant par exemple un service DICOMweb intitulé STOW (pour « STore Over the Web », en anglais), soit en mode « pull », à l'aide d'un service DICOMweb intitulé WADO (pour « Web Access to DICOM Object », en anglais). De façon alternative, les objets sont échangés via des services DIMSE (pour « DICOM Message Service Element », en anglais) intitulé C-Store en mode push et C-Move en mode pull, qui utilisent une connexion TCP/IP (pour « Transmission Control Protocol / Internet Protocol », en anglais).

Une fois que l'équipement NCSE a récupéré l'objet dé-identifié A', il le traite par exemple en lui appliquant un ou plusieurs traitements d'images d'un module MTI. On entend par traitement d'images toute opération sur une image numérique destinée à en améliorer la qualité ou à en extraire de l'information utile, par exemple au diagnostic d'une pathologie. Il peut s'agir par exemple d'un traitement de suivi de cible qui cherche à segmenter le contour d'un organe et à le suivre dans une séquence d'images.

On suppose qu'une image dérivée I' est obtenue. Elle comprend donc un résultat du ou des traitements réalisés. Par exemple, cette image I' comprend le contour segmenté d'un organe ou une prédiction de localisation d'une tumeur cancéreuse. Elle est encapsulée dans un nouvel objet conteneur B', puis retransmise vers le site clinique à destination de l'équipement CSE, par l'intermédiaire du réseau RT et à l'aide d'un des services précédemment évoqués.

Cet objet B' comprend des métadonnées identifiantes parmi lesquelles un identifiant d'image ID_{B'} (SOPInstanceUID) qui lui est propre. Il comprend aussi des métadonnées MR_{A'} héritées de l'objet A' d'entrée, que le module de traitement d'images MTI est généralement agencé pour recopier dans les champs correspondants de la séquence SINC de l'objet B'. Il s'agit notamment des champs « Nom du patient » comprenant la valeur factice « John Doe », « identifiant d'étude » comprenant la valeur factice « 2.25.237892542171 » et « identifiant de séries » comprenant la valeur factice « 2.25.5679909786302 ». On notera que l'identifiant ID_{A'} de l'image d'origine I dans l'objet dé-identifié A' n'est généralement pas recopié dans l'objet B' au cours de cette opération.

A son retour dans le site clinique, l'objet B' comprenant l'image dérivée I' doit être ré-identifié en vue d'être rattaché au dossier patient concerné.

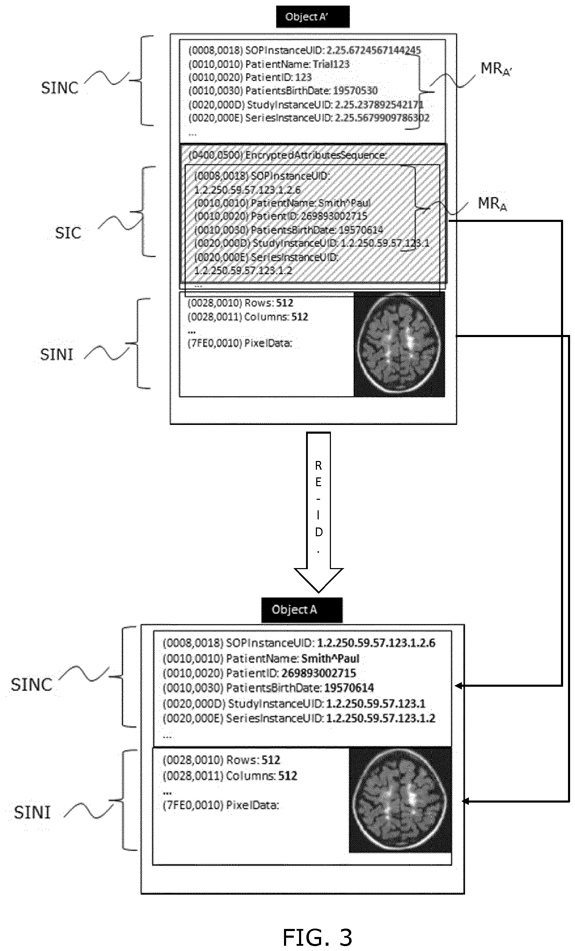

En relation avec la Figure **3****,** on a représenté de façon schématique une opération de ré-identification de l'objet dé-identifié A' par le site clinique, connue de l'homme de métier. Elle consiste à déchiffrer les métadonnées identifiantes MR_{A} stockées dans le champ SIC_{A'} et à les recopier dans la séquence SINC qui leur est réservé.

En revanche, l'application de cette opération de ré-identification à l'objet dérivé B' pose problème, du fait que sa séquence de champs d'informations chiffrées SIC_{B'} est vide et ne comprend donc pas de métadonnée identifiante chiffrée associée à l'image d'origine I. En effet, cette séquence ne peut être créée que par le site clinique qui ne connaît pas l'objet dérivé B'.

En effet, un module de traitement d'images MTI n'est généralement pas agencé pour conserver les métadonnées chiffrées contenues dans l'objet dé-identifié d'entrée A' et les recopier dans l'objet de sortie B'. En outre, dans le cas où le ferait, il créerait un objet incohérent du fait que l'identifiant d'image chiffré (SOPInstanceUID) serait celui de l'image d'origine I et ne correspondrait pas à l'image dérivée I'. Il en résulterait une ré-identification erronée de l'objet A au lieu de l'objet B, et donc un remplacement de l'objet principal A par l'objet dérivé B côté site cliniques, avec des risques importants pour le patient.

Face à ce problème, le standard DICOM a proposé, dans sa section PS3-15 E.3.9, une solution qui consiste à considérer que certaines métadonnées comme l'identifiant d'étude, de séquence d'images ou d'image ne sont pas à proprement parler identifiantes et qu'elles peuvent être maintenues en clair dans l'objet A' dé-identifié. De la sorte, et pourvu que le module de traitement d'image puisse les propager, l'objet de sortie B' comprenant l'image dérivée en hérite, ce qui permet ensuite de rattacher l'image dérivée à l'image d'origine lors de la ré-identification côté site clinique.

Néanmoins cette solution n'est pas satisfaisante en termes d'anonymisation car les identifiants d'étude, de séquence d'images ou d'image, identiques entre le site clinique et le site de recherche, permettent à un utilisateur averti sans être expert de retrouver le patient concerné. Le standard DICOM a décrit ces risques mais les estime limités.

Avec les avancées de la recherche translationnelle, qui consiste à donner accès au clinicien en charge du suivi d'un patient, à des résultats de recherche produits à partir des données de ce patient, ce problème de rattachement de l'objet conteneur d'une image dérivée d'une image d'origine aux données du patient se pose de plus en plus fréquemment.

### 3. Objectifs de l'invention

L'invention vient améliorer la situation.

L'invention a notamment pour objectif de pallier ces inconvénients de l'art antérieur.

Plus précisément, un objectif de l'invention est de proposer une solution qui permette à un site clinique de rattacher automatiquement un objet d'information médicale créé en dehors de ce site, aux données d'un patient, tout en respectant la sécurité des informations personnelles transférées et en restant conforme à un format d'objet d'information médical standardisé.

### 4. Exposé de l'invention

Ces objectifs, ainsi que d'autres qui apparaîtront par la suite, sont atteints à l'aide d'un procédé de ré-identification d'un objet d'informations médicales, dit objet dérivé, reçu par un équipement d'un site clinique en provenance d'un équipement d'un site non clinique via un réseau de communication, ledit objet comprenant une image dérivée d'une image d'origine et au moins une première métadonnée de rattachement d'un objet dé-identifié comprenant l'image d'origine, ledit procédé comprenant les étapes suivantes :
- Obtention de la au moins une première métadonnée dans un champ d'informations non chiffré de l'objet dérivé;
- Obtention d'au moins une deuxième métadonnée de rattachement d'un objet principal comprenant l'image d'origine, ladite au moins une deuxième métadonnée étant contenue dans un champ d'information chiffré correspondant d'un deuxième objet d'informations médicales dé-identifié comprenant en outre la au moins une première métadonnée dans un champ d'informations non chiffrées;
- Création d'un objet dérivé ré-identifié comprenant ladite image dérivée, à partir de la au moins une deuxième métadonnée obtenue.

L'invention propose une ré-identification d'un objet conteneur d'une image dérivée de l'image d'origine, issu d'un environnement non clinique anonymisé. Elle est mise en œuvre par un équipement d'un site clinique, qui récupère l'association entre au moins une première métadonnée d'identification associée à l'image d'origine en dehors du site clinique et contenue dans l'objet dérivé reçu et au moins une deuxième métadonnée d'identification associée à l'image d'origine dans le site clinique et disponible sous forme chiffrée. Selon l'invention, cette association est stockée dans un deuxième objet conteneur, issu du site clinique, et accessible à l'équipement du site clinique sous forme dé-identifiée.

L'invention s'appuie sur une approche tout-à-fait nouvelle et inventive de la ré-identification, qui consiste à ré-identifier une image dérivée d'une image d'origine, à partir des métadonnées d'identification stockées dans deux objets conteneurs distincts.

Contrairement à l'art antérieur, qui prend le parti de ne pas chiffrer certaines métadonnées d'identification de l'image d'origine dans l'objet dé-identifié transmis au site non clinique, l'invention rattache automatiquement une image dérivée reçue à un dossier patient sans modifier le format des objets conteneurs et sans compromis sur la sécurité des données personnelles.

Selon un aspect de l'invention, l'étape de création de l'objet dérivé ré-identifié comprend :
- Un déchiffrement de la au moins une deuxième métadonnée obtenue;
- Une recopie de la au moins une deuxième métadonnée déchiffrée dans un champ d'information non chiffré de l'objet dérivé correspondant à celui de la au moins une première métadonnée ; et
- Une recopie de l'image dérivée dans l'objet dérivé ré-identifié.

De cette manière, on reconstruit un objet d'informations médicales conforme aux objets créés par le site clinique.

Selon un aspect de l'invention, l'étape d'obtention d'au moins une deuxième métadonnée comprend une réception du deuxième objet d'informations médicales dé-identifié en provenance de l'équipement du site non clinique et une comparaison de métadonnées contenues dans des champs d'informations non chiffrés du deuxième objet d'informations médicales dé-identifié avec la au moins une première métadonnée, la deuxième au moins une métadonnée étant obtenue, lorsqu'une correspondance entre la au moins une première métadonnée et la au moins une deuxième métadonnée est trouvée.

Selon cette première option, les autres objets d'informations médicales transmises par le même site non clinique sont examinés pour rechercher s'ils contiennent la au moins une métadonnée associée à l'image dérivée. Si c'est le cas, les champs chiffrés sont déchiffrés pour extraire la au moins une deuxième métadonnée permettant d'identifier l'image d'origine. Par exemple, le deuxième objet peut être transmis en mode push par le site non clinique, selon une requête de type « STOW » telle que spécifiée par la norme DICOM.

Un avantage est que le site clinique reçoit directement, sans action volontaire de sa part, les deux objets nécessaires à la ré-identification de l'image dérivée.

Selon une deuxième option, l'étape d'obtention comprend en outre l'émission d'une requête de transmission d'un deuxième objet d'informations médicales dé-identifié à l'équipement de communication du site non clinique, la requête comprenant la au moins une première métadonnée.

Selon une variante, c'est le site clinique qui requiert en mode « pull » l'émission par le site non clinique d'un deuxième objet médical dé-identifié comprenant la au moins une première métadonnée. La au moins une première métadonnée permet d'identifier l'objet demandé dans la requête émise.

Un avantage est que le site non clinique n'envoie le deuxième objet au site clinique que lorsqu'il en fait la demande, ce qui évite de générer du trafic inutile.

Selon une troisième option, l'étape d'obtention comprend l'émission d'une requête d'extraction de la au moins une deuxième métadonnée stockée dans un champ d'information chiffrée correspondant du deuxième objet d'information médicales, ladite requête comprenant la au moins une première métadonnée.

De la sorte, le site clinique ne requiert du site non clinique que la au moins une deuxième métadonnée chiffrée, plutôt que l'intégralité du deuxième objet. Un avantage est d'économiser de la bande passante en limitant la quantité de données échangées au minimum.

Selon un autre aspect de l'invention, le deuxième objet d'informations médicales dé-identifié comprend l'image d'origine.

Un avantage de ce mode de réalisation est sa simplicité de mise en œuvre. Il n'est pas créé d'objet conteneur supplémentaire pour stocker l'association entre les métadonnées de l'image d'origine. Le site non clinique garde en mémoire l'objet conteneur dé-identifié de l'image d'origine et le renvoie ou bien en extrait la au moins une deuxième métadonnée chiffrée.

Selon encore un autre aspect de l'invention, le deuxième objet d'informations médicales dé-identifié est un objet d'informations médicales, dit accessoire de l'objet principal, qui comprend au moins une métadonnée de référence à un identifiant de l'image d'origine dans un champ d'informations chiffrées et ne comprend pas d'image.

Ce type d'objet d'informations médicales sert à transporter un pointeur sur une image ou instance d'une séquence d'images stockée dans un autre objet, afin de l'étiqueter ou d'y associer des commentaires. Selon la norme DICOM par exemple, il s'agit d'un objet de type « Sélection d'objet Clé » ou KOS (pour « Key Object Selection », en anglais). Un premier avantage est qu'il ne comprend pas de données image (pour « pixel data », en anglais) et évite de ce fait un deuxième transfert inutile de l'image d'origine entre les deux sites ou bien, dans le cas de l'extraction, la nécessité pour le site non clinique de stocker l'objet conteneur de l'image d'origine une fois l'image dérivée produite.

Un deuxième avantage est qu'il comprend nativement une référence à l'image d'origine dans son objet conteneur. Cette référence peut être mise à profit, dans un mode de réalisation particulier pour rattacher l'image dérivée directement et automatiquement à l'objet conteneur de l'image d'origine.

Selon une variante, cet objet comprend plusieurs références à des images d'une même séquence ou série d'images ou même à plusieurs séquences d'une même série. Ces références sont alors stockées dans des champs d'informations chiffrées distincts, avantageusement dans l'ordre de la séquence d'images. Un avantage est que ce seul et même objet accessoire est porteur de l'association de métadonnées nécessaire à la ré-identification de toutes les images dérivées des images d'origine issue de la séquence. De la sorte, les ressources de transmission et stockage sont économisées. Selon encore un autre aspect de l'invention, l'objet dérivé comprend en outre une métadonnée comprenant une référence à un identifiant de l'image d'origine dans l'objet principal dé-identifié, le procédé comprend l'obtention d'un identifiant de l'image d'origine dans un champ d'information chiffré correspondant du deuxième objet, l'étape de ré-identification de l'objet dérivé comprend le déchiffrement de ladite métadonnée et la recopie de la métadonnée déchiffrée dans un champ de référence à l'image d'origine dans l'objet dérivé (B) ré-identifié.

Ce mode de réalisation s'applique dans le cas où le traitement d'image appliqué à l'image d'origine par le site non clinique recopie l'identifiant de l'image d'origine dans l'objet dé-identifié A' dans un champ de référence de l'objet dérivé qu'elle produit. Cette métadonnée supplémentaire permet la récupération de l'identifiant de l'image d'origine stocké sous forme chiffrée dans le deuxième objet. Un avantage est que l'image dérivée peut être directement et automatiquement rattachée à l'image d'origine.

Selon encore un autre aspect de l'invention, l'étape d'obtention d'au moins une deuxième métadonnée comprend l'obtention d'une séquence de métadonnées chiffrées, l'étape de déchiffrement déchiffre au moins une deuxième métadonnée dite supplémentaire d'un champ de ladite séquence qui ne correspond pas à un champ d'une dite première métadonnées (MR_{B'}) et l'étape de recopie ladite deuxième métadonnée supplémentaire dans le champ correspondant non chiffré de l'objet dérivé ré-identifié lorsque au moins une première métadonnée extraite de l'objet dérivé reçu comprend un lien d'inclusion qui la rattache à ladite deuxième métadonnée supplémentaire.

Les métadonnées de rattachement s'incluent entre elles. Par exemple un identifiant ou un nom de patient peut donner lieu à une ou plusieurs études, une étude inclut une ou plusieurs séries lesquelles incluent une ou plusieurs images ou instances. L'invention exploite ce lien d'inclusion pour « récupérer » des métadonnées de rattachement supplémentaires de l'objet dérivé ré-identifié, qui ont pu être perdues lors du traitement réalisé par le site non clinique. Toutefois, pour éviter toute erreur qui pourrait être dommageable au patient, elle n'ajoute dans l'objet dérivé ré-identifié que les métadonnées déchiffrées issues du deuxième objet qui incluent une métadonnée présente dans l'objet dérivé B' reçu, comme par exemple le nom du patient, lorsqu'on dispose de l'identifiant d'étude. Un avantage est l'enrichissement des métadonnées de l'objet dérivé ré-identifié.

L'invention concerne également un dispositif de ré-identification d'un objet d'informations médicales dé-identifié, dit objet dérivé dé-identifié, reçu par un équipement de communication d'un site clinique en provenance d'un équipement d'un site non clinique via un réseau de communication, ledit objet comprenant une image dérivée d'une image d'origine et au moins une première métadonnée de rattachement d'un objet d'information médicales dé-identifié dit objet principal dé-identifié comprenant l'image d'origine dans un champ d'informations non chiffré, ledit dispositif étant caractérisé en ce qu'il comprend une machine de calcul dédiée à ou configurée pour :
- Obtenir la au moins une première métadonnée dans le champ d'informations non chiffré de l'objet dérivé dé-identifié ;
- Obtenir au moins une deuxième métadonnée de rattachement d'un objet principal comprenant l'image d'origine, ladite au moins une deuxième métadonnée étant contenue dans un champ d'information chiffrée d'un deuxième objet d'informations médicales dé-identifié comprenant la au moins une première métadonnée dans un champ d'informations non chiffrées ;
- Création d'un objet dérivé comprenant ladite image dérivée à partir de la au moins une deuxième métadonnée obtenue.

Ce dispositif est adapté pour mettre en œuvre le procédé de ré-identification d'un objet dérivé selon l'un quelconque des modes particuliers de réalisation définis ci-dessus. Il pourra bien sûr comporter les différentes caractéristiques relatives au procédé selon l'invention. Ainsi, les caractéristiques et avantages de ce dispositif sont les mêmes que ceux du procédé de ré-identification, et ne sont pas détaillés plus amplement.

Selon un mode particulier de réalisation de l'invention, un tel dispositif est compris dans un équipement du site clinique, par exemple un ordinateur ou PC (pour « personal computer », en anglais), une tablette, un téléphone intelligent (pour « smartphone », en anglais) ou tout autre équipement terminal disposant de moyens de connexion au site clinique.

L'invention concerne aussi un procédé de dé-identification d'un objet d'informations médicales, dit objet principal par un équipement de communication d'un site clinique en vue de sa transmission à un équipement d'un site non clinique via un réseau de communication, ledit objet comprenant une image d'origine et au moins une première métadonnée identifiante dans au moins un champ d'informations non chiffrées, ledit procédé comprenant les étapes suivantes :
- Création d'un objet principal dé-identifiécomprenant l'image d'origine, ladite création comprenant :
   ∘ le chiffrement de la au moins une première métadonnée et le stockage de la au moins une première métadonnée chiffrée dans au moins un champ d'information chiffrée dudit objet ;
   ∘ l'obtention d'au moins une deuxième métadonnée distincte de la première métadonnée et son stockage dans le au moins un champ d'informations non chiffré de l'objet principal dé-identifié, à la place de la au moins une première métadonnée ;
- Création d'un objet d'informations médicales, dit objet accessoire de l'objet principal, comprenant la au moins une première métadonnée de rattachement de l'objet principal dans un champ d'information non chiffrée, au moins une métadonnée de référence à l'image d'origine dans un champ d'information non chiffrée et ne comprenant pas d'image ;
- Création d'un objet accessoire dé-identifié, ladite création comprenant :
   ∘ Le chiffrement de la au moins une métadonnée de référence ;
   ∘ La recopie de la au moins une métadonnée de référence chiffrée dans un champ d'information chiffrée correspondant de l'objet accessoire dé-identifié ;
   ∘ La recopie de la au moins une première métadonnée chiffrée de l'objet principal dé-identifié dans au moins un champ d'information chiffré de l'objet accessoire dé-identifié ; et
   ∘ La recopie de la au moins une deuxième métadonnée dans le au moins un champ d'information non chiffrée correspondant de l'objet accessoire dé-identifié.

L'invention propose ainsi une dé-identification d'un objet principal conteneur) d'une image d'origine qui maintient une association entre les premières métadonnées identifiantes associées l'image d'origine dans l'objet principal A et des deuxièmes métadonnées, de valeurs factices, qui les remplacent dans l'objet principal dé-identifié.

Dans ce mode de réalisation de l'invention, cette association est stockée dans un objet conteneur supplémentaire, accessoire de celui de l'image d'origine, qui respecte les contraintes de format normatives. Ce conteneur ne contient pas d'image, il est donc de petite taille et peu coûteux à transmettre vers le site clinique.

Ce deuxième conteneur anonymisé est avantageusement transmis avec celui de l'image d'origine vers l'équipement de destination du site non clinique, de façon à maintenir l'association de métadonnées à proximité du conteneur dé-identifié de l'image d'origine. Il permet d'éviter un stockage local de cette association au niveau du site clinique, qui n'est pas autorisé pour des raisons de confidentialité. En particulier, le site clinique n'est pas autorisé à stocker une copie de l'objet conteneur dé-identifié de l'image d'origine qu'il a transmis à un site non clinique.

L'invention s'appuie donc sur une approche tout-à-fait nouvelle et inventive de la dé-identification d'informations médicales, qui va à l'encontre de l'art antérieur, pour permettre de préserver le lien entre les métadonnées associées à l'image dans le conteneur d'origine et dans le conteneur anonymisé, sans compromis sur la protection des données personnelles ni modification du format normatif du conteneur d'images.

Selon un aspect de l'invention, la au moins une deuxième métadonnée identifiante est stockée dans une mémoire locale suite à l'étape de dé-identification de l'objet principal.

De cette manière, on dispose de la ou les valeurs de deuxième métadonnée factice lors de la dé-identification de l'objet accessoire (KOS').

De façon alternative, la au moins une deuxième métadonnée est obtenue par application d'une fonction de hashage à la au moins une première métadonnée identifiante et cette fonction est appliquée au cours des étapes de dé-identification respectives de l'objet principal et de l'objet accessoire.

Un avantage est qu'il n'est pas nécessaire de stocker les deuxièmes métadonnées entre les deux étapes de création, puisque la fonction de hashage garantit d'obtenir la reproductibilité de l'opération.

De façon correspondante, l'invention concerne également un dispositif de dé-identification d'un objet d'informations médicales, dit objet principal par un équipement d'un site clinique en vue de sa transmission à un équipement d'un site non clinique via un réseau de communication, ledit objet comprenant une image d'origine et au moins une première métadonnée associée, ledit dispositif comprenant une machine de calcul dédiée à ou configurée pour :
- Créer un objet principal dé-identifié comprenant :
   ∘ le chiffrement de la au moins une première métadonnée identifiante et le stockage de la au moins une première métadonnée chiffrée dans au moins un champ d'information dudit objet ;
   ∘ la recopie de la au moins une deuxième métadonnée dans le au moins un champ d'informations non chiffré de l'objet principal dé-identifié ;
   ∘ le stockage de la au moins une image dans l'objet principal dé-identifié ;
- Créer un objet d'informations médicales, dit accessoire de l'objet principal, comprenant la au moins une première métadonnée identifiante de l'objet principal dans un champ d'information non chiffrée et ne comprenant pas d'image ;
- Créer un objet accessoire dé-identifié, comprenant :
   ∘ chiffrer la au moins une métadonnée de référence ;
   ∘ recopier la au moins une métadonnée de référence chiffrée dans un champ d'information chiffrée correspondant de l'objet accessoire dé-identifié ;
   ∘ recopier la au moins une première métadonnée chiffrée de l'objet principal dé-identifié dans au moins un champ d'information chiffré de l'objet accessoire dé-identifié ; et
   ∘ recopier la au moins une deuxième métadonnée dans le au moins un champ d'information non chiffrée correspondant de l'objet accessoire dé-identifié.

Ce dispositif est adapté pour mettre en œuvre le procédé de dé-identification d'un objet principal selon l'invention tel que défini ci-dessus. Il pourra bien sûr comporter les différentes caractéristiques relatives au procédé selon l'invention. Ainsi, les caractéristiques et avantages de ce dispositif sont les mêmes que ceux du procédé de dé-identification, et ne sont pas détaillés plus amplement. Selon un mode particulier de réalisation de l'invention, un tel dispositif est compris dans un équipement de communication du site clinique, par exemple un ordinateur ou PC (pour « personal computer », en anglais), une tablette, un téléphone intelligent (pour « smartphone », en anglais) ou tout autre équipement terminal disposant de moyens de connexion au site clinique.

Selon l'invention, un tel équipement de communication est apte à émettre et recevoir un objet d'information médicales dans ou d'un réseau de communication et il comprend le dispositif de ré-identification d'un objet d'informations médicales qui vient d'être décrit. Avantageusement, il comprend en outre un dispositif de dé-identification d'un objet d'informations médicales selon l'invention qui vient d'être décrite.

L'invention concerne aussi un programme d'ordinateur comportant des instructions pour la mise en œuvre des étapes d'un procédé de ré-identification d'un objet d'informations médicales tel que décrit précédemment, lorsque ce programme est exécuté par un processeur.

L'invention concerne aussi un programme d'ordinateur comportant des instructions pour la mise en œuvre des étapes d'un procédé de dé-identification d'un objet d'informations médicales tel que décrit précédemment, lorsque ce programme est exécuté par un processeur.

Ces programmes peuvent utiliser n'importe quel langage de programmation. Ils peuvent être téléchargés depuis un réseau de communication et/ou enregistrés sur un support lisible par ordinateur.

L'invention se rapporte enfin à des supports d'enregistrement, lisible par un processeur, intégrés ou non au dispositif de ré-identification et au dispositif de dé-identification selon l'invention, éventuellement amovible, mémorisant un programme d'ordinateur mettant en œuvre les procédés de ré-identification et de dé-identification selon l'invention, tels que décrits précédemment.

### 5. Liste des figures

D'autres avantages et caractéristiques de l'invention apparaîtront plus clairement à la lecture de la description suivante d'un mode de réalisation particulier de l'invention, donné à titre de simple exemple illustratif et non limitatif, et des dessins annexés, parmi lesquels :
- la figure **1** décrit de façon schématique une opération de dé-identification d'un objet d'informations médicales A en un objet d'informations médicales A' dé-identifié selon l'art antérieur ;
- la figure **2** illustre de façon schématique les échanges entre un équipement de communication d'un site clinique et un équipement de communication d'un site non clinique selon l'art antérieur ;
- la figure **3** décrit de façon schématique une opération de ré-identification d'un objet d'informations médicales A' en un objet d'informations médicales A selon l'art antérieur ;
- la figure **4** illustre de façon schématique les étapes d'un procédé de ré-identification d'un objet d'informations médicales dé-identifié dérivé, selon l'invention ;
- les figures **5** à **7** détaillent de façon schématique l'étape d'obtention d'une association entre au moins une métadonnée de rattachement extraite de l'objet dérivé et une métadonnée de rattachement chiffrée à partir d'un deuxième objet médical dé-identifié selon trois variantes de réalisation de l'invention ;
- la figure **8** illustre de façon schématique un objet d'informations médicales accessoire d'un autre objet médical ;
- la figure **9** décrit les étapes d'un procédé de dé-identification d'un objet d'informations médicales selon un premier mode de réalisation de l'invention ;
- la figure **10** illustre un exemple de dé-identification selon ce premier mode de réalisation de l'invention ;
- la figure **11** illustre un exemple de ré-identification d'un objet dérivé selon ce premier mode de réalisation de l'invention, lorsque le deuxième objet est l'objet accessoire KOS' ;
- la figure **12** illustre un exemple de ré-identification d'un objet dérivé selon ce premier mode de réalisation de l'invention, lorsque le deuxième objet est l'objet principal A' ;
- la figure **13** décrit le procédé de ré-identification d'un objet d'informations médicales selon un deuxième mode de réalisation de l'invention ;
- la figure **14** illustre un exemple de ré-identification d'un objet dérivé selon ce deuxième mode de réalisation de l'invention, lorsque le deuxième objet est l'objet principal A';
- la figure **15** illustre un exemple de ré-identification d'un objet dérivé selon ce deuxième mode de réalisation lorsque le deuxième objet est l'objet accessoire KOS'; et
- la figure **16** décrit de façon schématique la structure matérielle d'un dispositif de dé-identification d'un objet d'informations médicales et la structure matérielle d'un dispositif de ré-identification d'un objet d'informations médicales selon un mode de réalisation de l'invention.

### 6. Description d'un mode de réalisation particulier de l'invention

L'invention concerne la ré-identification par un site clinique d'un objet d'informations médicales, reçu d'un site non clinique et comprenant une image dérivée d'une image d'origine, cette image d'origine ayant été initialement transférée par le site clinique au site non clinique dans un objet d'informations médicales dé-identifié.

Le principe de l'invention repose sur l'utilisation d'une association de métadonnées contenue dans un deuxième objet d'informations médicales, lui aussi disponible au niveau du site non clinique.

Dans la suite, on désigne par champs de métadonnées correspondants des champs qui ne sont pas forcément désignés par le même nom ou intitulé, mais sont sensiblement équivalents en ce qu'ils contiennent des métadonnées de même type. Par exemple, on considère qu'un champ d'identification d'une image dans un objet, intitulé par exemple « SOPInstanceUID » selon la norme DICOM, correspond à un champ de référence à l'identification d'une image dans un objet, intitulé par exemple « ReferencedSOPIntanceUID », selon la même norme.

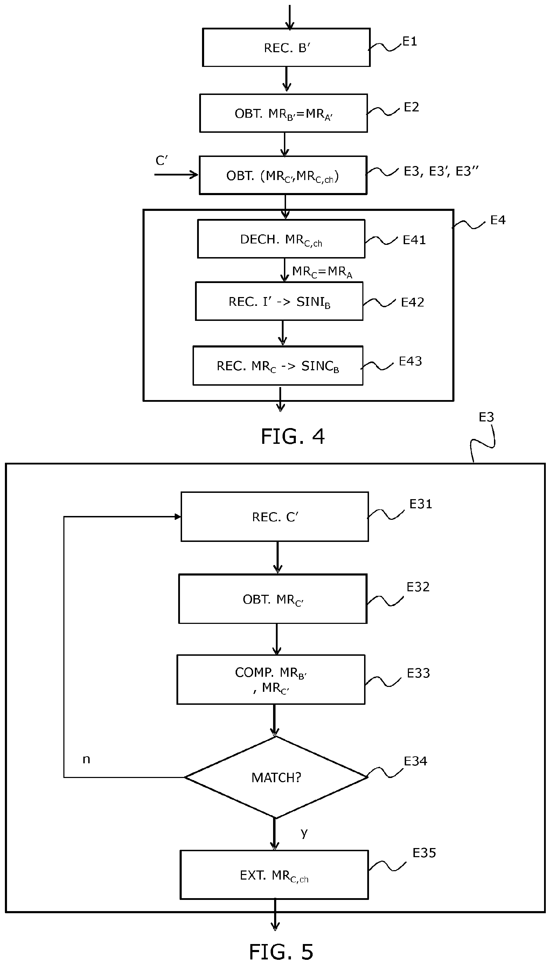

En relation avec la Figure **4**, on décrit les étapes d'un procédé de ré-identification d'un objet dérivé B' selon un premier mode de réalisation de l'invention. Ce procédé est destiné à être mis en œuvre suite à l'obtention de l'objet dérivé B' par un équipement de communication CSE du site clinique. Au cours d'une étape E1, l'objet dérivé B' est obtenu. Il est reçu d'un équipement de communication NCSE du site non clinique. Cet objet a été créé par le site non clinique pour contenir une image dérivée I'. Il ne comprend pas de métadonnées chiffrées car il n'a pas subi de dé-identification côté site clinique. Cette réception déclenche en E2 une lecture de la séquence de champs d'informations non chiffrées SINC_{B'} de l'objet B', qui comprend notamment au moins une première métadonnée de rattachement MR_{B'} telle que le nom du patient ou sa date de naissance ou encore un identifiant d'étude et/ou un identifiant de séries dans cette étude. On rappelle que cette séquence comprend nécessairement une métadonnée d'identification ID_{B'} de l'image dérivée I' contenue dans l'objet B', qu'elle est contenue dans un champ d'identification d'image (pour « SOPInstanceUID", en anglais), et qu'elle n'est pas considérée comme une métadonnée de rattachement, car elle est propre à l'image dérivée I'.

En E3, on obtient une association entre la au moins une première métadonnée de rattachement et au moins une deuxième métadonnée de rattachement chiffrée MR_{2,ch}, ladite association étant contenue dans un deuxième objet d'informations médicales dé-identifié disponible au niveau du site non clinique.

En E4, on créé un objet dérivé ré-identifié comprenant l'image dérivée, à partir de la au moins une deuxième métadonnée de rattachement obtenue.

Plusieurs variantes de réalisation de cette étape d'obtention E3 sont envisagées. Elles vont maintenant être présentées en relation avec les Figures **5** à **7**.

Selon une première variante, en relation avec la Figure **5****,** l'étape d'obtention E3 comprend une réception E31 d'un deuxième objet d'informations médicales C' en provenance de l'équipement NCSE. En E32, la séquence de champs non chiffrés SINC de l'objet C' est lue et au moins une métadonnées de rattachement MR_{C'} qu'elle contient est extraite. En E33, la au moins une métadonnée de rattachement MR_{C'} est comparée à la au moins une métadonnée de rattachement MR_{B'} de l'objet dérivé B' à ré-identifier.

Avantageusement, chacune des premières métadonnées MR_{B'} de l'objet B' est comparée à toutes les métadonnées MR_{C'} de l'objet C' dans le but de trouver une correspondance.

Si une correspondance est établie en E34, alors on considère que l'objet C' et l'objet B' sont liés à un même dossier patient et on va lire la ou les métadonnées de rattachement chiffrées MR_{C,ch} contenues dans la séquence de champs chiffrés SIC de l'objet C'. En E35, elles sont déchiffrées. Ces métadonnées de rattachement déchiffrées MR_{C'} sont de nature à identifier un patient, une étude et/ou encore une série associée à l'image d'origine I, à partir de laquelle l'image dérivée I' a été obtenue.

Sinon, si aucune correspondance n'a pu être établie, alors on se met en attente de réception d'un autre objet médical dé-identifié en provenance de l'équipement de communication NCSE.

On notera que si l'objet B' ne comprend qu'une première métadonnée MR_{B'} ou qu'une sous-partie des métadonnées de la séquence SINC_{B'}, alors la précision du rattachement dépend du type des métadonnées disponibles. Par exemple, si une seule métadonnée MR_{B'} est disponible et qu'il s'agit de l'identifiant de la série d'images, alors le rattachement de l'image dérivée I' sera précis. En revanche, si la seule métadonnée disponible est l'identifiant d'étude, on pourra remonter à l'étude dont est issue l'image d'origine I mais on ne peut pas garantir que le rattachement à la série identifiée par la métadonnée correspondante du deuxième objet soit correct.

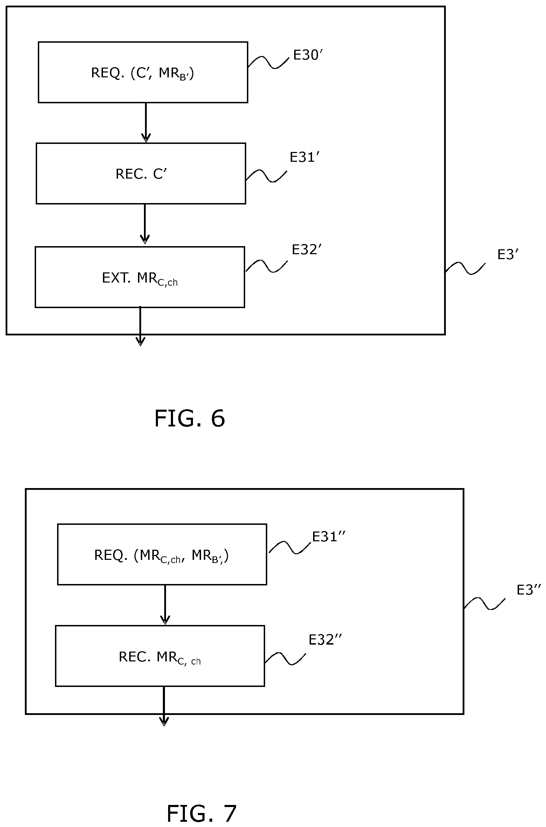

Selon une deuxième variante, en relation avec la Figure **6****,** l'obtention comprend l'émission d'une requête E30' d'obtention d'un deuxième objet d'informations médicales C' à destination de l'équipement de communication NCSE. Cette requête comprend au moins une métadonnée de rattachement MR_{B'} extraites de l'objet B'. En réponse, un deuxième objet dé-identifié C' est reçu en E31'. Il comprend dans sa séquence SIN_{C'} la au moins une métadonnée MR_{B'} transmise dans la requête. En E32', la au moins une métadonnée de rattachement chiffrée correspondante contenue dans la séquence de champs SIC_{C'} est lue puis déchiffrée en E4. Ce mode de réalisation correspond à la mise en œuvre d'une transmission en mode pull, par exemple à l'aide d'un message de type WADO tel que spécifié par la norme DICOM.

On notera que, selon cette deuxième variante, si l'objet B' ne comprenait qu'une seule métadonnée de rattachement, par exemple le nom du patient, ou une sous-partie des métadonnées de rattachement de la séquence SINC, alors la réponse à la requête pourrait comprendre plusieurs objets C' au sein desquels il conviendrait d'en choisir un. Dans un tel cas, seules les métadonnées relatives au « patient », telles que son identifiant, son nom ou sa date de naissance pourront être correctement initialisées, à partir des métadonnées d'un de ces objets C' et une solution pour obtenir un objet B conformément ré-identifié serait de créer une nouvelle étude, à laquelle le rattacher.

Selon une troisième variante, en relation avec la Figure **7****,** l'obtention comprend l'émission E31" d'une requête d'obtention d'au moins une métadonnée de rattachement chiffrée associée à au moins une métadonnée de rattachement MR_{B'} extraite de l'objet dérivé B'. Avantageusement, elle comprend au moins le type de la métadonnée de rattachement comprenant l'identifiant de l'étude à laquelle se rapporte l'image d'origine I et dont dérive l'image I'. Sur réception en E31" d'au moins une métadonnée chiffrée MR_{C,ch} répondant à la requête, elle est transmise à l'étape E4 de déchiffrement. Ce mode de réalisation correspond à la mise en œuvre d'une transmission sélective, d'une ou plusieurs métadonnées plutôt que d'un objet entier, en mode pull, par exemple à l'aide d'un message de type WADO sélectif, tel que spécifié par la norme DICOM.

Les métadonnées chiffrées MR_{C,ch} reçues comprennent au moins la métadonnée chiffrée de même type que celle spécifiée dans la requête. Toutefois, on notera que cette requête d'extraction partielle de l'objet C', permet généralement d'extraire l'intégralité de sa séquence chiffrée SIC_{C'}.

Dans l'exemple de la Figure 4, l'étape E4 comprend en E1 un déchiffrement de la ou les métadonnées MR_{C'} obtenues. Dans le cas où l'intégralité de la séquence SIC_{C'} est déchiffrée, au moins la métadonnée correspondant à celle requise est extraite de la séquence déchiffrée. Dans le cas où il s'agit de l'identifiant de l'étude, d'autres métadonnées de rattachement, telles que l'identifiant du patient, son nom ou sa date de naissance peuvent avantageusement être extraites pour être ensuite ajoutées à l'objet ré-identifié B. En effet, il existe un lien d'inclusion entre ces métadonnées, qui garantit que l'identifiant d'étude ne peut être rattaché qu'à un seul identifiant de patient. De la sorte, si jamais l'identifiant de patient n'a pas été reporté par le module de traitement de l'image d'origine I dans l'objet dérivé créé B', il peut être récupéré lors de sa ré-identification, sans risque d'erreur de rattachement. Au contraire, si l'identifiant de série n'est pas disponible dans les métadonnées MR_{B'}, l'identifiant de série déchiffré issu de la séquence SIC_{C'} ne doit pas être recopié dans l'objet dérivé ré-identifié B, car l'étude en question peut inclure plusieurs séries et on ne peut pas garantir que l'identifiant de série obtenu corresponde à la série dont est réellement issue l'image d'origine I.

En E42, les métadonnées déchiffrées extraites sont recopiées dans les champs correspondants de la séquence de métadonnées non chiffrées SINC_{B} de ce nouvel objet. L'image dérivée I' est copiée en E43 dans le champ correspondant du nouvel objet B. On obtient ainsi un objet dérivé B comprenant l'image dérivée I' qui est rattaché par au moins une métadonnée MR_{C'} au dossier patient dont est issue l'image d'origine I.

En outre, si le site non clinique dispose en mémoire de plusieurs objets C' comprenant la au moins une métadonnée de rattachement MR_{A'} spécifiée dans la requête, alors plusieurs options sont envisagées. Selon une première option, la réponse comprend un des objets C' ou la séquence de métadonnées chiffrées SIC_{C'} d'un des objets C' répondant à la requête, ce qui permet de récupérer au moins la ou les métadonnées chiffrées correspondant aux métadonnées de rattachement MRA' requises. Selon une deuxième option, tous les objets C' répondant à la requête ou bien toutes les séquences chiffrées sont transmises dans la réponse.

Selon l'invention, le deuxième objet C' qui vient d'être présenté, porteur de l'association entre au moins une métadonnée de rattachement non chiffrée de l'objet dérivé B' et au moins une métadonnée de rattachement chiffrée MR_{A,ch} de l'objet principal A', peut-être de différents types.

Selon une première variante, le deuxième objet C' est l'objet A'. Un avantage est que cet objet A' est le résultat de la dé-identification de l'objet A comprenant l'image d'origine I. Il est nécessairement transmis au site non clinique en vue du traitement de l'image I et comprend dans sa séquence de champs chiffrés SIC toutes les métadonnées de rattachement de l'image I, telles que l'identifiant d'étude et l'identifiant de série auxquelles l'image d'origine appartient.

Selon une deuxième variante, le deuxième objet C' est un objet, dit accessoire KOS', distinct de A', qui ne contient pas d'image, mais au moins une référence ou pointeur sur l'image d'origine I de l'objet principal A. Par exemple, cet objet est de type « Sélection d'Objet Clé » (pour « Key Object Selection », en anglais), comme spécifié dans la norme DICOM (PS3-3 « A.35.4 Key Object Selection Document IOD »). La référence à l'image I de l'objet A est stockée dans une métadonnée de référence à une instance, intitulée « ReferencedSOPInstanceUID », dans un champ d'information chiffrée de la séquence SIC_{KOS'}. Ce format d'objet a été prévu par la norme pour étiqueter ou « flagger » une ou plusieurs images d'une même série voire d'une même étude. Selon l'invention, son usage est détourné pour stocker l'association entre les métadonnées de rattachement non chiffrées de l'objet B' issues de l'objet A' et les métadonnées de rattachement correspondantes chiffrées de l'objet A', issues de l'objet A.

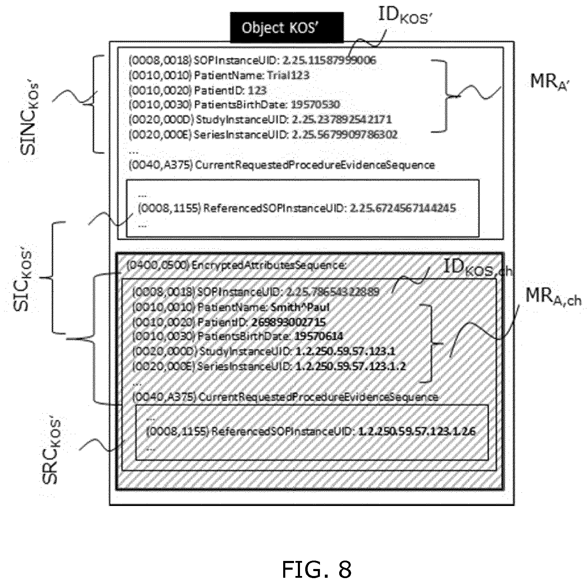

En relation avec la Figure **8****,** on a représenté un exemple d'objet accessoire KOS' selon l'invention. Il comprend une telle association entre A' et A. Un avantage de cet objet accessoire est sa petite taille, du fait qu'il ne contient pas d'image. Ceci présente un intérêt particulier dans les premier et deuxième modes de réalisation de l'invention, selon lesquels le deuxième objet C' est transmis dans son intégralité du site non clinique au site clinique.

On notera que cet objet accessoire KOS' est issu du site clinique et qu'il a nécessairement été transmis par l'équipement CSE à l'équipement NCSE du site non clinique dans une étape préalable. Un avantage supplémentaire important est que ce type d'objet peut contenir de multiples références à d'autres objets, par exemple les références aux objets conteneurs de plusieurs images d'une même série ou de plusieurs séries d'une même étude, et que dans le cas, fréquent, où les opérations de dé-identification, traitement et ré-identification concernent toutes les images d'une série ou de plusieurs séries au sein d'une même étude, un seul objet KOS permet de gérer la ré-identification de l'ensemble des objets dérivés du traitement.

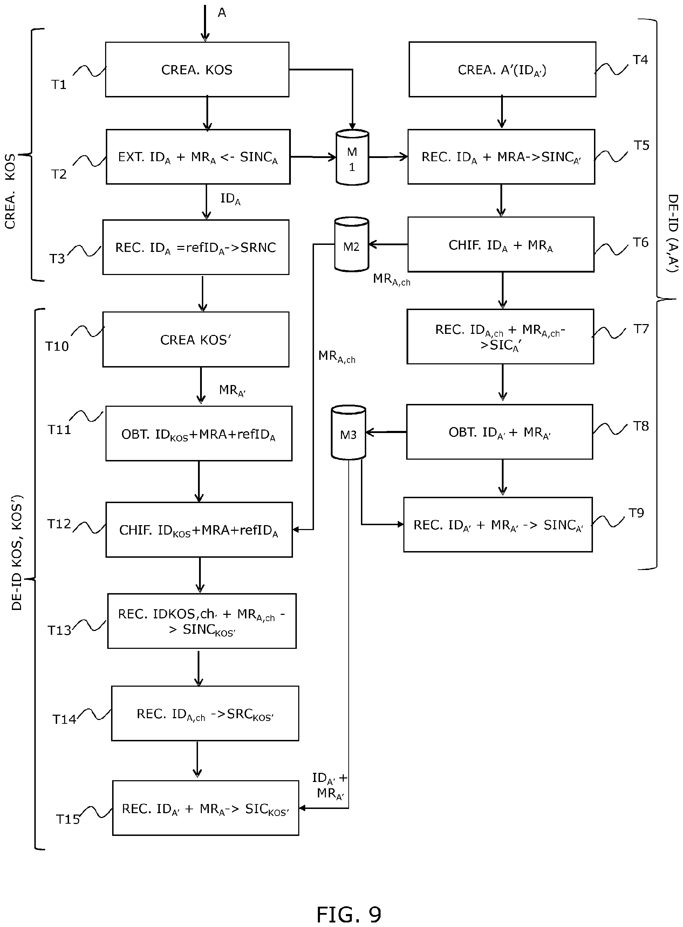

En relation avec la Figure **9****,** on décrit maintenant un procédé de dé-identification d'un objet principal porteur d'une image d'origine I selon cette deuxième variante du premier mode de réalisation de l'invention.

On dispose côté site clinique d'un objet principal A, comprenant l'image I stockée dans une séquence de champs de métadonnées non identifiantes SINI_{A}, un identifiant d'image I_{A} et au moins une métadonnée de rattachement MR_{A}.

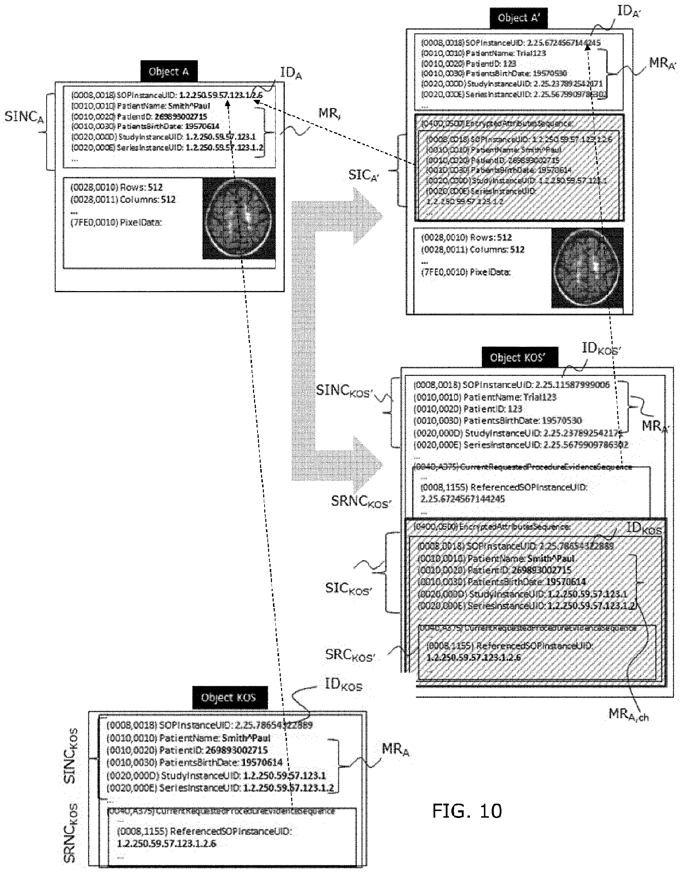

La Figure **10** illustre de façon schématique la structure de l'objet principal A et des autres objets A', KOS et KOS' créés à partir de l'objet principal A selon ce mode de réalisation de l'invention.

Une première phase de ce procédé englobant les étapes T1 à T3 concerne la création de l'objet accessoire KOS. En T1, on crée un nouvel objet KOS, dit accessoire de l'objet A. Il possède un identifiant d'image ID_{KOs} qui lui est propre. En T2, on extrait l'identifiant ID_{A} de l'image I dans l'objet A et les métadonnées de rattachement MR_{A} de l'objet principal A et on les stocke temporairement dans une mémoire locale M1. On recopie les métadonnées de rattachement MR_{A} dans les champs correspondants de la séquence SINC_{KOS} de l'objet KOS. En T3, on recopie l'identifiant d'image ID_{A} dans le champ de la métadonnée de référence à une instance de l'objet accessoire KOS, intitulé

« ReferencedSOPInstanceUID ». Par exemple, ce champ appartient à une séquence SRNC_{KOS} de champs de métadonnées de référence non chiffrées qui fait suite à la séquence SINC_{KOS}. On note que l'objet KOS ainsi créé ne contient pas d'image, mais comprend un pointeur sur l'image I de l'objet A.

Au cours d'une deuxième phase englobant les étapes T4 à T9, on réalise une dé-identification de l'objet A, connue de l'homme de métier. En T4, on crée un nouvel objet A'. Il comprend un identifiant d'image ID_{A'} qui lui est propre. En T5, on extrait de l'objet A ou bien on lit dans la mémoire M1, l'identifiant ID_{A} de l'image I dans l'objet A et les métadonnées de rattachement MR_{A} qui lui sont associées. En T6, elles sont chiffrées selon une méthode connue, par exemple basée sur une clé publique. Les métadonnées chiffrées ID_{A,ch} et MR_{A,ch} sont recopiées en T7 dans une séquence de champs chiffrés SIC_{A'} de l'objet A'. Avantageusement elles sont stockées temporairement dans une mémoire locale M2 pour un usage ultérieur. En T8, on obtient au moins une métadonnée de rattachement MR_{A'} pour l'objet dé-identifié A', de valeur factice. Par exemple, sa valeur est obtenue de façon aléatoire ou bien par le moyen d'une fonction de hashage, de façon connue de l'homme de métier et par exemple selon la technique SHA1 spécifiée dans le document RFC3174 de l'organisme de normalisation IETF (pour « Internet Engineering Task Force », en anglais), par Eastlake et al. en septembre 2001.

Dans les deux cas, le format de la valeur obtenue doit être conforme à celui de la métadonnée MR_{A'}. On note que dans le cas où la valeur de la métadonnée MR_{A'} est obtenue aléatoirement, elle est stockée temporairement dans une mémoire locale M3 pour un usage ultérieur, ce qui n'est pas nécessaire lorsqu'on a recours à une fonction de hashage. En T9, on recopie la ou les valeurs obtenues dans le ou les champs non chiffrés correspondants de la séquence SINC_{A'} de l'objet dé-identifié A'.

Au cours d'une troisième phase, on dé-identifie l'objet accessoire KOS. En T11, on crée un nouvel objet accessoire KOS'. Il possède un identifiant d'image I_{KOS'} qui lui est propre. En T12, on extrait l'identifiant d'image I_{KOS}, de l'objet KOS, les métadonnées de rattachement MR_{A} de la séquence SINC_{KOS} et la métadonnée RefID_{A}=ID_{A} de référence à l'image d'origine I de la séquence SRNC_{KOS} ou bien on les lit dans la mémoire M1 et on les chiffre à l'aide d'une clé publique, de façon connue en soi. De façon alternative, on peut aussi récupérer les métadonnées ID_{A,ch} et MR_{A,ch} déjà chiffrées au cours de l'étape T7, pourvu qu'elles aient été stockées par exemple dans la mémoire M2.

En T13, on recopie l'identifiant d'image chiffré I_{KOS,ch} et les métadonnées chiffrées MR_{A,ch} dans la séquence de champs chiffrés SIC_{KOS'} de l'objet accessoire KOS'. En T14, on recopie la métadonnée RéfID_{KOS' =}ID_{A,ch} dans un champ correspondant d'une séquence de métadonnées de référence chiffrées SRC_{KOS'}. En T15, on obtient les métadonnées de rattachement MR_{A'} stockées dans la séquence de champs non chiffrés SINC_{A'} de l'objet A' et on les recopie dans les champs correspondants de la séquence SINC_{KOS'}. Selon une première option, on va lire leurs valeurs directement dans l'objet dé-identifié A', ce qui implique que cet objet n'ait pas encore été transmis à l'équipement NCSE du site non clinique. En effet, cet objet ne peut pas être stocké en mémoire dans le site clinique qui n'a pas vocation à le faire pour des objets dé-identifiés, destinés à un environnement de recherche. Selon une deuxième option, les métadonnées MR_{A'} ont été temporairement stockées dans une mémoire M3 et sont lues directement dans cette mémoire. On note que, dans ce cas, elles seront effacées une fois que le procédé n'en aura plus besoin.

On obtient ainsi un objet dé-identifié KOS' qui contient une association entre des métadonnées de rattachement MR_{A} de l'objet A, stockées sous forme chiffrée dans la séquence de champs chiffrés SIC_{KOS'} et des métadonnées de rattachement MR_{A'} de l'objet A', stockées dans la séquence de champs non chiffrées SINC_{KOS'}. Il est donc à la fois accessoire des objets principaux A et A'.

En T16, les deux objets dé-identifiés A' et KOS' sont transmis à l'équipement de communication NCSE du site non clinique. On notera qu'ils peuvent être transmis indépendamment l'un de l'autre, de façon non simultanée.

Selon une variante, le site clinique prévoit de transmettre au site non clinique plusieurs images I₁, I₂,...,I_{N}, avec N entier supérieur ou égal à 2, de la même série d'images à laquelle appartient l'image I₁ = I. Dans ce cas, un seul objet KOS' peut être avantageusement exploité pour transporter l'association de métadonnées nécessaire à la ré-identification d'objets dérivés de A et d'autres images de la série, selon l'invention.

Le procédé qui vient d'être décrit est adapté de la façon suivante :
- L'objet accessoire KOS créé au cours de la première phase, inclut une référence réfID_{I1}, réfID_{I2}, ... réfID_{IN} à chacune des images I₁, I₂,...I_{N} dans la séquence SRNC_{KOS}. Par exemple, elles sont stockées les unes à la suite des autres selon l'ordre des images dans la séquence;
- Les objets A1, A2, AN sont dé-identifiés au cours de la deuxième phase comme déjà décrit en relation avec la Figure 9 ;
- L'objet accessoire KOS' est dé-identifié au cours de la troisième phase comme déjà décrit. Les références réfID_{I1}, réfID_{I2}, ... réfID_{IN} sont chiffrées et recopiées dans les champs correspondants de la séquence SRC_{KOS'}. Les identifiants ID_{A'1}, ID_{A'2}...ID_{A'N} sont recopiés dans les champs correspondants de la séquence de métadonnées non chiffrées SRNC_{KOS'}. Les métadonnées MR_{A'} étant communes à toutes les images de la séquence, l'étape T9 de recopie de ces métadonnées de l'objet A' dans la séquence de métadonnées non chiffrées SINC_{KOS'} est inchangée.

L'objet dé-identifié KOS' et les N objets dé-identifiés A_{1'},A_{2'}...A_{N'} peuvent être transmis ensemble ou séparément, en une ou plusieurs opérations de transfert, en utilisant par exemple le service STOW, qui a été évoqué précédemment.

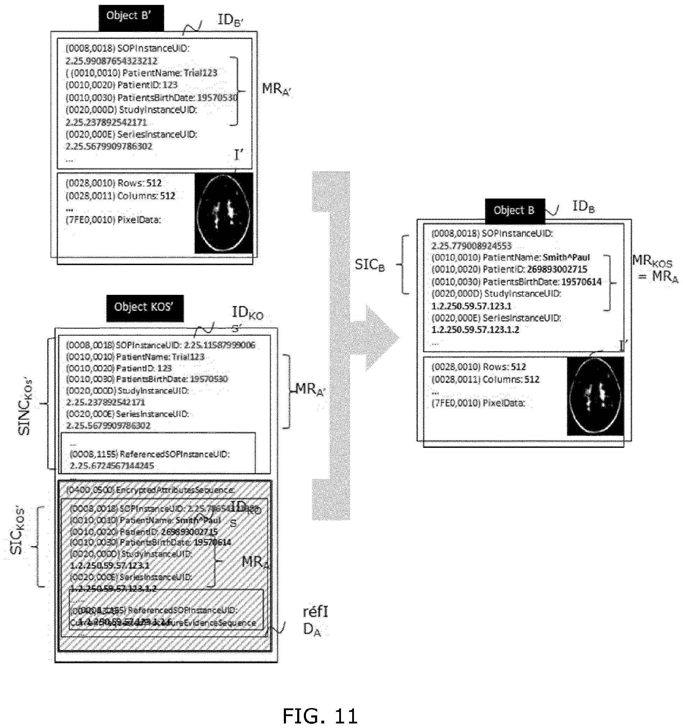

En relation avec la Figure **11****,** on détaille maintenant un premier exemple de mise en œuvre du procédé de ré-identification d'un objet B' dérivé de l'objet A', selon le premier mode de réalisation de l'invention et reçu par un équipement CSE du site clinique en provenance d'un équipement NCSE du site non clinique, décrit en relation avec les figures **4** à **7****.**

L'objet dérivé B' a été produit par un module de traitement d'images MTI du site non clinique. Il contient une image I' résultant de ce traitement. L'objet B' est reçu par l'équipement CSE du site clinique, ce qui déclenche la mise en œuvre du procédé de traitement selon l'invention, qui vise à rattacher l'image I' à un objet d'informations médicales d'un patient hébergé par le site clinique. Plus précisément, il s'agit de ré-identifier l'objet B' en créant un objet B comprenant l'image dérivée I' et des métadonnées de nature à rattacher l'image I au dossier patient dont est issue l'image I.

Selon l'invention, ce rattachement est réalisé à partir d'un deuxième objet, dit C', disponible au niveau du site non clinique. Comme déjà évoqué en relation avec les figures **4** à **7****,** ce deuxième objet C' peut être effectivement reçu par l'équipement CSE ou bien il est rendu disponible côté site non clinique et l'équipement CSE requiert l'extraction de certaines métadonnées qu'il contient. Dans l'exemple de la Figure **11****,** on considère que cet objet C' est l'objet accessoire dé-identifié KOS' précédemment décrit en relation avec la figure **8** et qu'il a été reçu par l'équipement CSE.

Les métadonnées de rattachement MR_{B'} stockées dans la séquence non chiffrée SINC_{B'} de l'objet B' sont lues.

Dans cet exemple, elles comprennent un identifiant d'étude, un identifiant de série, un identifiant de patient, un nom de patient et une date de naissance du patient.

L'équipement CSE extrait les métadonnées MP_{KOS'} stockées dans la séquence non chiffrée SINC_{KOS'} de l'objet KOS' et les compare aux métadonnées MR_{B'}. Si elles correspondent, c'est-à-dire si les valeurs des métadonnées MR_{B'} se retrouvent dans les champs correspondants MR_{KOS'}, alors l'objet KOS' est considéré comme accessoire de l'objet A issu du site clinique et contenant l'image d'origine I à partir de laquelle l'image I' a été produite. Un déchiffrement des métadonnées stockées dans la séquence de champs chiffrés SIC_{KOS'} de l'objet KOS' est alors mis en œuvre. Il s'appuie sur une technique connue en soi qui repose sur l'utilisation d'une clé privée associée à la clé publique utilisée pour chiffrer les métadonnées, par exemple selon la méthode AES (pour « Advance Encryption Standard », en anglais) décrite dans le document RFC3268 de Chown, publié en juin 2002, par l'IETF.

Les métadonnées déchiffrées comprennent un identifiant ID_{A} de l'image dérivée I issue de l'objet A et des métadonnées MR_{KOS'}. Selon l'invention, ces métadonnées contiennent les valeurs des métadonnées MR_{A} associées à l'image d'origine I et issues de l'objet principal A qui contient cette image. Un objet B est créé. Les métadonnées MR_{A} déchiffrées correspondant à celles présentes dans la séquence SINC_{B'} sont donc recopiées dans les champs correspondants de la séquence SIC_{B}. L'image I' est recopiée dans le champ SINI de l'objet B. L'identifiant ID_{A} identifie l'image d'origine I, mais il ne peut être associé à aucune métadonnée de l'objet dérivé B'. En effet le champ correspondant « SOPInstanceUID » de l'objet B' comprend un identifiant de l'image dérivée I' attribué par le site non clinique à l'objet B'. On ne peut donc pas savoir si l'image dérivée I' résulte du traitement de cette image I ou d'une autre image de la série. Il en résulte qu'on ne peut pas exploiter cette métadonnée ID_{A}.

En revanche, vu qu'on on dispose ici de l'identifiant de série, l'invention permet de rattacher l'image I' directement et automatiquement à la série dont est issue l'image I.

On comprend que ce rattachement est le plus précis qui puisse être obtenu à partir des métadonnées de rattachement MR_{A} obtenues dans cet exemple.

Comme déjà évoqué, dans le cas où l'identifiant d'étude est présent dans les métadonnées de rattachement MR_{B'}, mais pas l'identifiant de série, l'invention rattache l'image dérivée de l'objet B' à l'étude identifiée par l'identifiant d'étude chiffré de l'objet C', de façon à éviter toute erreur.

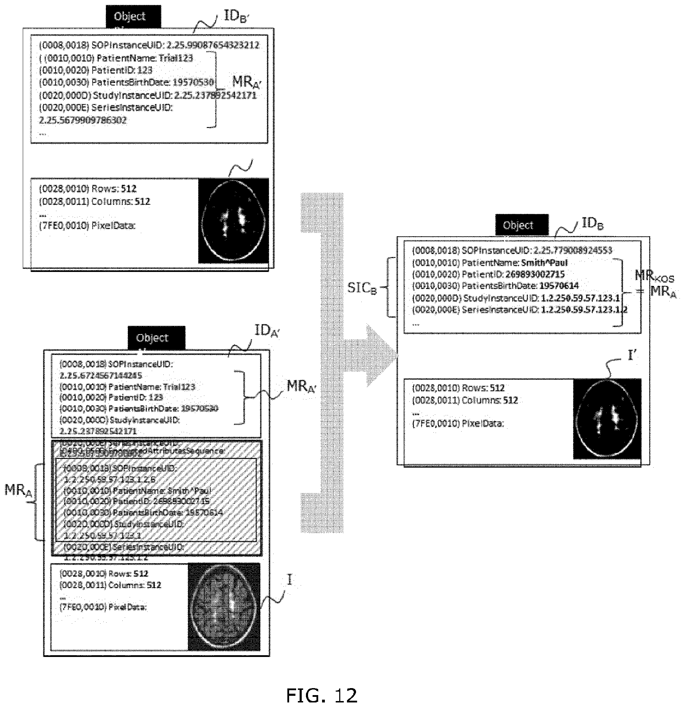

La Figure **12** illustre un deuxième exemple de mise en œuvre du procédé de ré-identification d'un objet B' dérivé de l'objet A' à partir d'un objet C' qui correspond à l'objet A'. Comme précédemment décrit, l'objet A' associe par nature des premières métadonnées de rattachement non chiffrées MR_{A'} à des deuxièmes métadonnées de rattachement chiffrées MR_{A}. Le procédé de ré-identification selon l'invention s'applique de façon similaire au cas précédent et ne sera pas décrit plus avant. De façon similaire, si l'identifiant de série IDS_{A} est une métadonnée de rattachement disponible dans l'objet dérivé B', alors l'invention permet un rattachement direct et automatique de l'objet B ré-identifié à la série dont est issue l'image I.

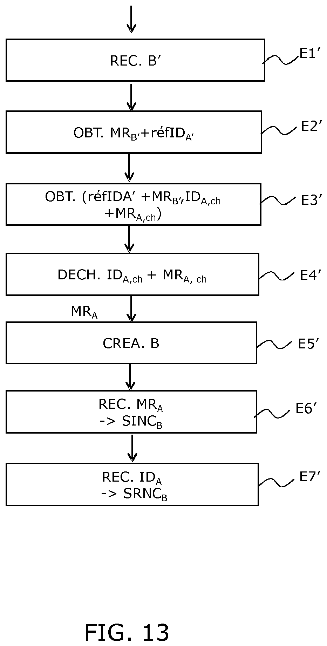

En relation avec les Figures **13****,** **14** et **15****,** on décrit maintenant un deuxième mode de réalisation de l'invention. On suppose maintenant que le module de traitement d'image MTI du site non clinique est agencé pour stocker, dans un champ de l'objet dérivé B', la valeur de la métadonnée ID_{A'} d'identification de l'image d'origine I dans l'objet dé-identifié A'.

Par exemple, cette valeur, qu'on désignera par réfID_{A'} est stockée dans un champ de référence à un identifiant d'image de l'objet dérivé B', par exemple intitulé « ReferencedSOPInstanceUID » dans la norme DICOM.

En relation avec la Figure **13****,** sur réception de l'objet B', l'équipement de communication CSE du site clinique, déclenche la mise en œuvre du procédé de traitement qui vient d'être décrit en relation avec les Figures **4** à **7**. Selon ce deuxième mode de réalisation, l'étape E' est similaire à l'étape E1 déjà décrite. L'étape E2' d'obtention de métadonnées contenues dans l'objet dérivé B' comprend non seulement l'extraction déjà décrite des métadonnées MR_{B'} de la séquence SINC_{B'} de l'objet B', mais aussi celle de la métadonnée supplémentaire réfID_{A'} stockée dans le champ « ReferencedSOPInstanceUID » d'une séquence de métadonnées de référence non chiffrées SRNC_{B'}. Au cours de l'étape E3', le procédé de ré-identification obtient l'association entre les métadonnées de rattachement MR_{B'}, réfID_{A}, extraites et des métadonnées chiffrées MR_{A,ch} de l'objet A contenues dans des champs correspondants d'une séquence de champs chiffrés SICc' objet C'.

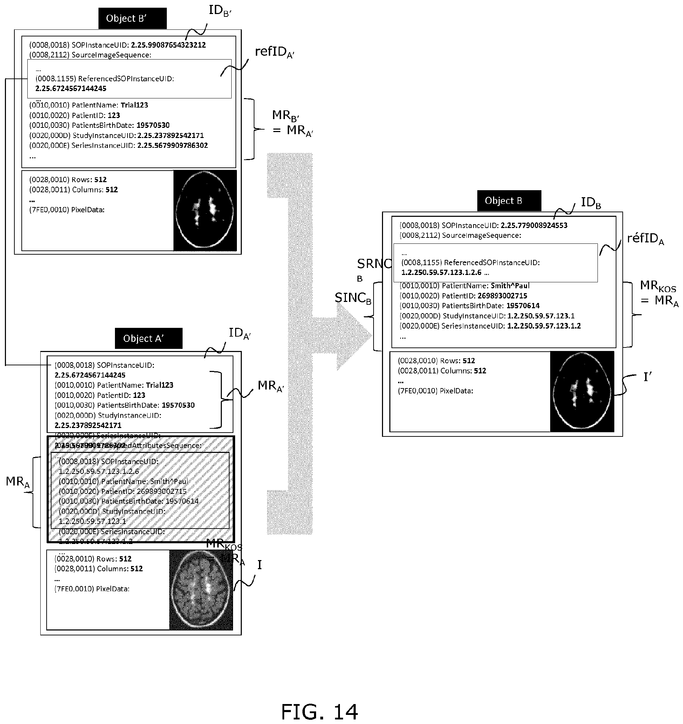

En relation avec la Figure **14****,** lorsque l'objet C' est l'objet A', alors la métadonnée supplémentaire réfID_{A}, de l'objet B' correspond à la valeur de la métadonnée stockée dans le champ non chiffré « SOPInstanceUID », qui comprend l'identifiant ID_{A'} de l'image I dans l'objet A'. Cette métadonnée supplémentaire de l'objet B' est donc avantageusement comparée en E2' aux métadonnées de champs correspondants de la séquence de champs SINC_{C'} de l'objet C' comme décrit en relation avec les Figures **5** et **6****,** ou encore spécifiée dans une requête d'extraction sélective des métadonnées chiffrées de l'objet A' comme décrit en relation avec la Figure **7**. En E3', on obtient les métadonnées chiffrées MR_{A, ch} stockées dans la séquence SIC_{A'} de l'objet A'. Dans ce cas, le champ correspondant à celui de la métadonnée supplémentaire réfID_{A'} est le champ SOPInstanceUID de la séquence chiffrée SIC_{A'} de l'objet A' qui comprend l'identifiant d'image d'origine ID_{A,ch} dans l'objet principal A. En E4', les métadonnées obtenues sont déchiffrées. En E5', l'objet B est créé avec un champ « ReferencedSOPInstanceUID ». En E6', les métadonnées MRA déchiffrées de même type que les métadonnées MR_{B'} sont recopiées dans les champs correspondants de la séquence SINC_{B}. En E7' l'identifiant IDA est recopié dans le champ « ReferencedSOPInstanceUID » de l'objet B.

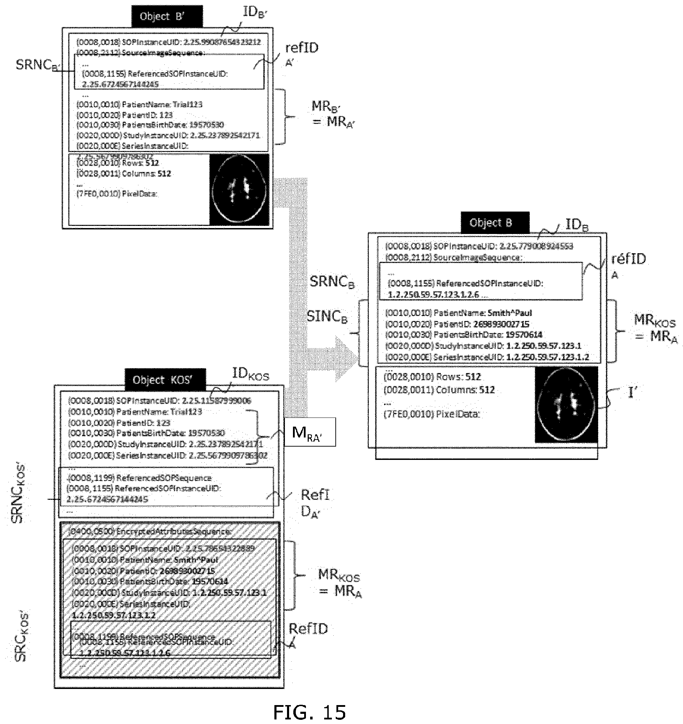

En relation avec la Figure **15****,** lorsque l'objet C' est l'objet KOS', la métadonnée supplémentaire réfID_{A}, extraite en E2' de l'objet dérivé B' ne trouve pas de correspondance en E3' avec la métadonnée stockée dans le champ non chiffré « SOPInstanceUID » de l'objet KOS', qui comprend dans ce cas l'identifiant ID_{KOS'} de l'image I' dans l'objet KOS', mais avec celle du champ non chiffré « ReferencedSOPInstanceUID » de la séquence de métadonnées de référence SRNC_{KOS'}.

La présence de cette métadonnées supplémentaire réfID_{A}, dans l'objet B' déclenche au niveau de l'étape E4' le déchiffrement du champ ID_{A} identifiant l'image A dans la séquence SIC_{KOS} de l'objet C', en plus des champs correspondants aux métadonnées de rattachement MR_{B'} issues de l'objet KOS'. Ainsi, suite à la création de l'objet B en E4, l'étape E5 recopie non seulement les métadonnées MR_{A} déchiffrées dans les champs correspondants de la séquence de champs non chiffrés SINC_{B} de l'objet B, mais aussi l'identifiant d'image d'origine ID_{A} à la place de la métadonnée réfID_{A'} dans le champ « referencedSOPInstanceUID » de la séquence SRNC_{B} de l'objet B. Les étapes E5' à E7' sont inchangées.

De la sorte, avec l'invention, l'objet dérivé porteur de l'image se trouve directement automatiquement rattaché à l'image d'origine I.

On notera que l'invention qui vient d'être décrite, peut être mise en œuvre au moyen de composants logiciels et/ou matériels. Dans cette optique, les termes « module » et « entité », utilisés dans ce document, peuvent correspondre soit à un composant logiciel, soit à un composant matériel, soit encore à un ensemble de composants matériels et/ou logiciels, aptes à mettre en œuvre la ou les fonctions décrites pour le module ou l'entité concerné(e).

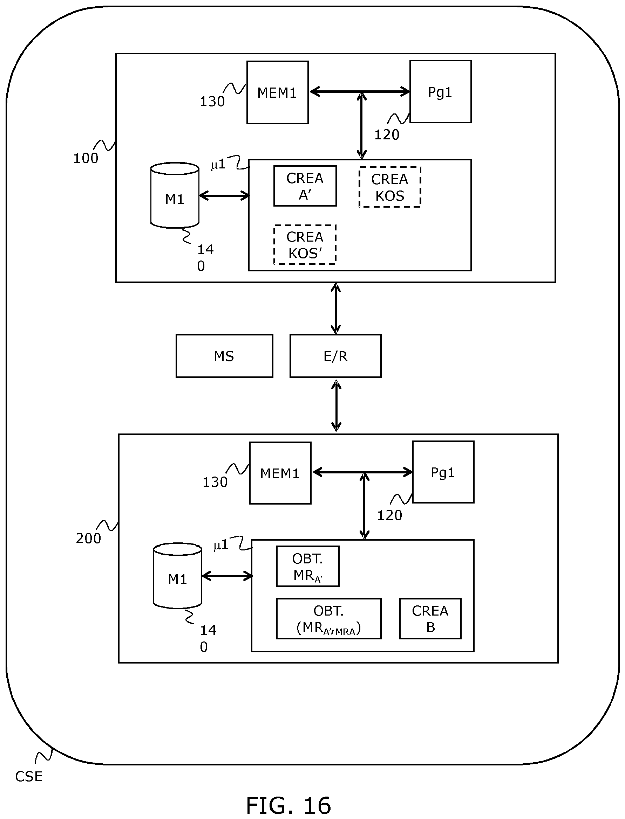

En relation avec la Figure **16****,** on présente maintenant un exemple de structure simplifiée d'un dispositif 100 de ré-identification d'un objet d'informations médicales comprenant une image dérivée d'une image d'origine selon l'invention. Le dispositif 100 met en œuvre le procédé de ré-identification d'un objet d'informations médicales dérivé selon l'invention qui vient d'être décrit en relation avec les figures 4 à 8.

Cette figure **16** illustre seulement une manière particulière, parmi plusieurs possibles, de réaliser les algorithmes détaillés ci-dessus. En effet, la technique de l'invention se réalise indifféremment sur une machine de calcul reprogrammable (un ordinateur PC, un processeur DSP ou un microcontrôleur) configurée pour exécuter un programme comprenant une séquence d'instructions, ou sur une machine de calcul dédiée (par exemple un ensemble de portes logiques comme un FPGA ou un ASIC, ou tout autre module matériel).

Dans le cas où l'invention est implantée sur une machine de calcul reprogrammable, le programme correspondant (c'est-à-dire la séquence d'instructions) pourra être stocké dans un médium de stockage amovible (tel que par exemple une disquette, un CD-ROM ou un DVD-ROM) ou non, ce médium de stockage étant lisible partiellement ou totalement par un ordinateur ou un processeur.

Par exemple, le dispositif 100 comprend une unité de traitement 110, équipée d'un processeur µ1, et pilotée par un programme d'ordinateur Pg1 120, stocké dans une mémoire 130 et mettant en œuvre le procédé de selon l'invention.

A l'initialisation, les instructions de code du programme d'ordinateur Pg₁ 120 sont par exemple chargées dans une mémoire RAM avant d'être exécutées par le processeur de l'unité de traitement 110. Le processeur de l'unité de traitement 110 met en œuvre les étapes du procédé décrit précédemment, selon les instructions du programme d'ordinateur 120.

Dans cet exemple de réalisation de l'invention, le dispositif 100 comprend une machine de calcul reprogrammable ou une machine de calcul dédiée, apte à et configurée pour :
- Obtenir OBT. MRA' la au moins une première métadonnée MR_{A'} dans le champ d'informations non chiffré de l'objet dérivé ;
- Obtenir OBT. MR_{A,ch} au moins une deuxième métadonnée MR_{A,ch} contenue dans un champ d'information chiffré (correspondant) d'un deuxième objet d'informations médicales dé-identifié A', KOS', ledit deuxième objet comprenant en outre la au moins une première métadonnée (MR_{A'}) dans un champ d'informations non chiffrées;
- Créer CREA. B un objet dérivé B comprenant l'image dérivée I', à partir de la au moins une deuxième métadonnée.

Avantageusement, la machine de calcul est configurée pour mettre en œuvre les modes de réalisation de l'invention qui viennent d'être décrits en relation avec les Figures **4** à **15****.**

En particulier, elle est en outre apte à mettre en œuvre les modes « pull » et « pull sélectif » décrits en relation avec les figures **6** et **7**. Elle est aussi apte à traiter n'importe quel type de deuxième objet, pourvu qu'il contienne l'association requise entre les première et deuxième métadonnées. Elle est enfin apte, lorsque l'objet dérivé dé-identifié B' comprend en outre une métadonnée comprenant une référence à un identifiant réfID_{A}, de l'image d'origine I, obtenir un identifiant de l'image d'origine ID_{A} dans un champ d'information chiffré du deuxième objet, à déchiffrer ladite métadonnée et à recopier la métadonnée déchiffrée dans un champ de référence à l'image d'origine de l'objet dérivé B créé.

Le dispositif 100 comprend en outre une unité M₁ 140 de stockage, telle qu'une mémoire, par exemple de type mémoire tampon (pour « buffer », en anglais), apte à stocker par exemple les deuxièmes métadonnées MR_{A'} dont les valeurs factices sont recopiées dans les champs non chiffrés de la séquence SINC de l'objet dé-identifié A', en vue de leur utilisation pour la dé-identification de l'objet KOS, accessoire de l'objet A.

Ces unités sont pilotées par le processeur µ1 de l'unité de traitement 110.

De façon avantageuse, un tel dispositif 100 peut être intégré à l'équipement de communication CSE du site clinique ou à tout autre équipement connecté à ce dernier au sein du site clinique.

Le dispositif 100 est alors agencé pour coopérer au moins avec les modules suivants de l'équipement de communication CSE :
- un module E/R d'émission/réception de données, par l'intermédiaire duquel un objet d'informations médicales dé-identifié est reçu à ou transmis d'un site non clinique; et
- un module de stockage MS d'objets d'informations médicales, tels que par exemple l'objet principal A.

L'équipement de communication CSE comprend en outre un dispositif 200 de dé-identification d'un objet d'informations médicales comprenant une image d'origine, dont on présente maintenant un exemple de structure simplifiée.

Dans le cas où l'invention récupère l'association entre les métadonnées de rattachement de l'objet A et de l'objet A' dans l'objet A', le dispositif 200 met en œuvre un procédé de dé-identification classique, connu de l'homme de métier.

Dans le cas où l'invention récupère cette association dans un objet accessoire KOS', le dispositif 200 met en œuvre le procédé de dé-identification selon l'invention qui vient d'être décrit en relation avec la figure **11****.**

Par exemple, le dispositif 200 comprend une unité de traitement 210, équipée d'un processeur µ2, et pilotée par un programme d'ordinateur Pg2 220, stocké dans une mémoire 230 et mettant en œuvre le procédé de selon l'invention.

A l'initialisation, les instructions de code du programme d'ordinateur Pg₁ 220 sont par exemple chargées dans une mémoire RAM avant d'être exécutées par le processeur de l'unité de traitement 210. Le processeur de l'unité de traitement 210 met en œuvre les étapes du procédé décrit précédemment, selon les instructions du programme d'ordinateur 220.

Dans cet exemple de réalisation de l'invention, le dispositif 200 comprend une machine de calcul reprogrammable ou une machine de calcul dédiée, apte à et configurée pour :
- Créer un objet principal dé-identifié A' comprenant l'image d'origine I, comprenant :
   ∘ Chiffrer la au moins une première métadonnée MR_{A} et stocker la au moins une première métadonnée chiffrée MR_{A,ch} dans au moins un champ d'information chiffrée SIC_{A'} dudit objet ;
   ∘ Obtenir au moins une deuxième métadonnée MR_{A'} distincte de la première métadonnée et la stocker dans le au moins un champ d'informations non chiffré SINC_{A'} de l'objet dé-identifié A', à la place de la au moins une première métadonnée MR_{A} ;
- Créer un objet d'information médicale KOS, dit accessoire de l'objet principal A, comprenant la au moins une première métadonnée identifiante MR_{A})de l'objet principal A et ne comprenant pas d'image ;
- Créer un objet accessoire dé-identifié KOS', ladite création comprenant :
   ∘ Le chiffrement de la au moins une métadonnée de référence réfID_{A} ;
   ∘ La recopie de la au moins une métadonnée de référence chiffrée dans un champ d'information chiffrée SRC_{KOS'} correspondant de l'objet accessoire dé-identifié KOS';
   ∘ La recopie de la au moins une première métadonnée chiffrée MR_{A,ch} de l'objet principal dé-identifié A' dans au moins un champ d'information chiffré SIC_{KOS'} de l'objet accessoire dé-identifié KOS'; et
   ∘ La recopie de la au moins une deuxième métadonnée MR_{A'} dans le au moins un champ d'information non chiffrée correspondant SINC_{KOS'} de l'objet accessoire dé-identifié KOS'.

Le dispositif 200 comprend en outre une unité M₂ 240 de stockage, telle qu'une mémoire, par exemple de type mémoire tampon (pour « buffer », en anglais), apte à stocker par exemple la au moins une deuxième métadonnée MR_{A'} dont les valeurs factices sont recopiées dans les champs non chiffrés de la séquence SINC de l'objet dé-identifié KOS', et la au moins une première métadonnée chiffrée MR_{A,ch} de l'objet principal dé-identifié A'.

Ces unités sont pilotées par le processeur µ2 de l'unité de traitement 210.

De façon avantageuse, un tel dispositif 200 peut être intégré à l'équipement de communication CSE du site clinique ou à tout autre équipement connecté à ce dernier au sein du site clinique.

Le dispositif 200 est alors agencé pour coopérer au moins avec les modules suivants de l'équipement de communication CSE :
- un module E/R d'émission/réception de données, par l'intermédiaire duquel un objet d'informations médicales dé-identifié est reçu à ou transmis d'un site non clinique; et
- un module de stockage MS d'objets d'informations médicales, tels que par exemple l'objet principal A ou l'objet dérivé B.

Grâce à ses bonnes performances et à sa simplicité de mise en œuvre, l'invention qui vient d'être décrite facilite le rattachement automatique à un dossier patient d'un site clinique d'une image dérivée d'un traitement effectué en dehors de ce site sur une image d'origine anonymisée de ce patient. Elle garantit un échange interopérable et sécurisé des informations de santé entre le site clinique et le site non clinique, qui respecte les libertés fondamentales des patients.

Il va de soi que les modes de réalisation qui ont été décrits ci-dessus ont été donnés à titre purement indicatif et nullement limitatif, et que de nombreuses modifications peuvent être facilement apportées par l'homme de l'art sans pour autant sortir du cadre de l'invention.

## Revendications

1. Procédé de ré-identification d'un objet d'informations médicales, dit objet dérivé dé-identifié (B'), reçu par un équipement (CSE) d'un site clinique en provenance d'un équipement (NCSE) d'un site non clinique via un réseau de communication (RT), ledit objet (B') comprenant une image (I') dérivée d'une image d'origine (I) et au moins une première métadonnée (MR_{A'}) de rattachement d'un objet d'informations médicales, dit objet principal dé-identifié (A') comprenant l'image d'origine (I), ledit procédé étant **caractérisé en ce qu'**il comprend les étapes suivantes :
- Obtention (E2) de la au moins une première métadonnée (MR_{A'}) dans un champ d'informations non chiffré (SINC_{B'}) de l'objet dérivé dé-identifié (B') ;
- Obtention (E3) d'au moins une deuxième métadonnée (MR_{A,ch}) de rattachement d'un objet principal (A) comprenant l'image d'origine, ladite au moins une deuxième métadonnée étant contenue dans un champ d'information chiffré (SIC_{C'}) correspondant d'un deuxième objet d'informations médicales dé-identifié (C', A', KOS'), ledit deuxième objet comprenant en outre la au moins une première métadonnée (MR_{A'}) dans un champ d'informations non chiffrées (SINC_{C'});
- Création (E4) d'un objet dérivé (B), comprenant ladite image dérivée (I') à partir de la au moins une deuxième métadonnée (MR_{A,ch}) obtenue.

2. Procédé de ré-identification d'un objet d'informations médicales, selon la revendication 1, **caractérisé en ce que** l'étape de création comprend :
∘ Un déchiffrement (E41) de la au moins une deuxième métadonnée (MR_{A,ch}) obtenue;
∘ Une recopie (E42) de la au moins une deuxième métadonnée déchiffrée (MR_{A}) dans un champ d'information non chiffré (SINC_{B}) de l'objet dérivé ré-identifié (B) correspondant à celui de la au moins une première métadonnée; et
∘ Une recopie (E43) de l'image dérivée (I') dans l'objet dérivé (B).

3. Procédé de ré-identification selon l'une des revendications **1** ou **2, caractérisé en ce que** l'étape d'obtention (E3) d'au moins une deuxième métadonnée comprend une réception (E31) du deuxième objet d'informations médicales dé-identifé (C', A', KOS') en provenance de l'équipement d'un site non clinique (NCSE) et une comparaison (E32) de métadonnées contenues dans des champs d'informations non chiffrés du deuxième objet d'informations médicales dé-identifié avec la au moins une première métadonnée (MR_{A'}), la deuxième au moins une métadonnée (MR_{A}, _{ch}) étant obtenue, lorsqu'une correspondance entre la au moins une première métadonnée et la au moins une deuxième métadonnée est trouvée.

4. Procédé de ré-identification selon la revendication **3, caractérisé en ce que** l'étape d'obtention (E3') comprend en outre l'émission (E30) d'une requête de transmission du deuxième objet d'informations médicales dé-identifié (C', A', KOS') à l'équipement de communication (NCSE), la requête comprenant la au moins une première métadonnée (MR_{A'}).

5. Procédé de ré-identification selon l'une des revendications **1** ou **2, caractérisé en ce que** l'étape d'obtention (E3") comprend l'émission (E31") d'une requête d'extraction de la au moins une deuxième métadonnée d'un champ d'informations chiffrées du deuxième objet d'information médicales dé-identifié (A', KOS'), ladite requête comprenant la au moins une première métadonnée (MR_{A'}).

6. Procédé de ré-identification selon l'une des revendications précédentes, **caractérisé en ce que** le deuxième objet d'informations médicales dé-identifié (A') comprend l'image d'origine (I).

7. Procédé de ré-identification selon l'une des revendications **1** à **4, caractérisé en ce que** le deuxième objet d'informations médicales dé-identifié est un objet d'informations médicales (KOS'), dit objet accessoire de l'objet principal dé-identifié (A'), qui comprend au moins une métadonnée de référence à l'image d'origine (réfID_{A}) dans un champ d'informations chiffrées et ne comprend pas d'image.

8. Procédé de ré-identification selon l'une des revendications précédentes, **caractérisé en ce que**, l'objet dérivé dé-identifié (B') comprenant en outre une métadonnée comprenant une référence (réfID_{A'}) à un identifiant (ID_{A'}) de l'image d'origine (I) dans l'objet principal dé-identifié (A'), le procédé comprend l'obtention (E2') d'un identifiant de l'image d'origine (ID_{A}) dans un champ d'information chiffré (SINC_{C'}) correspondant du deuxième objet, l'étape de ré-identification de l'objet dérivé dé-identifié (B') comprend le déchiffrement (E4') de ladite métadonnée et la recopie (E6') de la métadonnée déchiffrée dans un champ de référence (réfID_{A}) à l'image d'origine dans l'objet dérivé (B)ré-identifié.

9. Procédé de ré-identification selon l'une des revendications précédentes, **caractérisé en ce que**, l'étape d'obtention (E3) comprend l'obtention d'une séquence de deuxièmes métadonnées chiffrées (SIC_{C'}), l'étape de déchiffrement (E4) déchiffre au moins une deuxième métadonnée dite supplémentaire d'un champ de ladite séquence qui ne correspond pas à un champ d'une dite première métadonnées (MR_{B'}) et l'étape (E5) recopie ladite deuxième métadonnée supplémentaire dans le champ correspondant non chiffré de l'objet dérivé (B) lorsque au moins une première métadonnée extraite de l'objet dérivé dé-identifié (B') reçu comprend un lien d'inclusion qui la rattache à ladite deuxième métadonnée supplémentaire.

10. Dispositif (200) de ré-identification d'un objet d'informations médicales dé-identifié, dit objet dérivé dé-identifié (B'), reçu par un équipement de communication d'un site clinique (CSE) en provenance d'un équipement (NCSE) d'un site non clinique via un réseau de communication (RT), ledit objet comprenant une image dérivée (I') d'une image d'origine (I) et au moins une première métadonnée (MR_{A'}) de rattachement d'un objet d'information médicales dé-identifié dit objet principal dé-identifié (A') comprenant l'image d'origine (I) dans un champ d'informations non chiffré, ledit dispositif étant **caractérisé en ce qu'**il comprend une machine de calcul dédiée à ou configurée pour :
- Obtenir (OBT. MR_{A'}) la au moins une première métadonnée (MR_{A'}) dans le champ d'informations non chiffré de l'objet dérivé dé-identifié (B') ;
- Obtenir (OBT. MR_{A'}, MR_{A,ch}) au moins une deuxième métadonnée (MR_{A,ch}) de rattachement d'un objet principal (A) comprenant l'image d'origine, ladite au moins une deuxième métadonnée étant contenue dans un champ d'information chiffrée d'un deuxième objet d'informations médicales dé-identifié (C', A', KOS') comprenant la au moins une première métadonnée (MR_{A'}) dans un champ d'informations non chiffrées (SINC_{C'});
- Création (CREA. B) d'un objet dérivé (B) comprenant ladite image dérivée (I') à partir de la au moins une deuxième métadonnée (MR_{A,ch}) obtenue.

11. Procédé de dé-identification d'un objet d'informations médicales, dit objet principal (A), par un équipement de communication d'un site clinique (CSE) en vue de sa transmission à un équipement d'un site non clinique (RSE) via un réseau de communication, ledit objet comprenant une image d'origine (I) et au moins une première métadonnée de rattachement (MR_{A}) dans au moins un champ d'informations non chiffré (SINC_{A}), ledit procédé comprenant les étapes suivantes :
- Création d'un objet principal dé-identifié (A') comprenant l'image d'origine (I), ladite création comprenant :
∘ le chiffrement (T6) de la au moins une première métadonnée (MR_{A}) et le stockage (T7) de la au moins une première métadonnée chiffrée (MR_{A,ch}) dans au moins un champ d'information chiffrée (SIC_{A'}) dudit objet ;
∘ l'obtention (T8) d'au moins une deuxième métadonnée (MR_{A'}) distincte de la première métadonnée et son stockage dans le au moins un champ d'informations non chiffré (SINC_{A'}) de l'objet principal dé-identifié (A'), à la place de la au moins une première métadonnée (MR_{A}) ;
ledit procédé comprenant en outre les étapes suivantes :
- Création d'un objet d'informations médicales (KOS), dit objet accessoire de l'objet principal (A), comprenant la au moins une première métadonnée (MR_{A}) de rattachement de l'objet principal (A) dans un champ d'information non chiffrée (SINC_{KOS}), au moins une métadonnée de référence à l'image d'origine (réfID_{A}) dans un champ d'information non chiffrée (SRNC_{KOS}) et ne comprenant pas d'image ;
- Création d'un objet accessoire dé-identifié (KOS'), ladite création comprenant :
∘ Le chiffrement de la au moins une métadonnée de référence (réfID_{A}) ;
∘ La recopie de la au moins une métadonnée de référence chiffrée (réfID_{A,ch}) dans un champ d'information chiffrée (SRC_{KOS'}) correspondant de l'objet accessoire dé-identifié (KOS');
∘ La recopie de la au moins une première métadonnée chiffrée (MR_{A,ch}) de l'objet principal dé-identifié (A') dans au moins un champ d'information chiffré (SIC_{KOS'}) de l'objet accessoire dé-identifié (KOS'); et
∘ La recopie de la au moins une deuxième métadonnée (MR_{A'}) dans le au moins un champ d'information non chiffrée correspondant (SINC_{KOSO} de l'objet accessoire dé-identifié (KOS').

12. Dispositif (200) de dé-identification d'un objet d'informations médicales, dit objet principal (A) par un équipement d'un site clinique (CSE) en vue de sa transmission à un équipement d'un site non clinique (RSE) via un réseau de communication, ledit objet comprenant une image d'origine (I) et au moins une première métadonnée associée (MR_{A}), ledit dispositif comprenant une machine de calcul dédiée à ou configurée pour :
- Créer un objet principal dé-identifié (A') comprenant :
∘ le chiffrement de la au moins une première métadonnée identifiante (MR_{A}) et le stockage de la au moins une première métadonnée chiffrée (MR_{A,ch}) dans au moins un champ d'information (SIC_{A'}) dudit objet ;
∘ la recopie de la au moins une deuxième métadonnée (MR_{A'}) dans le au moins un champ d'informations non chiffré (SINCA') de l'objet principal dé-identifié (A') ;
∘ le stockage de la au moins une image (I) dans l'objet principal dé-identifié (A'); ledit dispositif est en outre apte à :
- Créer un objet d'informations médicales (KOS), dit accessoire de l'objet principal (A), comprenant la au moins une première métadonnée identifiante (MR_{A}) de l'objet principal (A) dans un champ d'information non chiffrée (SINC_{KOS}) et ne comprenant pas d'image ;
- Créer un objet accessoire dé-identifié (KOS'), comprenant :
∘ chiffrer la au moins une métadonnée de référence (réfID_{A}) ;
∘ recopier la au moins une métadonnée de référence chiffrée dans un champ d'information chiffrée (SRC_{KOS'}) correspondant de l'objet accessoire dé-identifié (KOS');
∘ recopier la au moins une première métadonnée chiffrée (MR_{A,ch}) de l'objet principal dé-identifié (A') dans au moins un champ d'information chiffré (SIC_{KOS'}) de l'objet accessoire dé-identifié (KOS'); et
∘ recopier la au moins une deuxième métadonnée (MR_{A'}) dans le au moins un champ d'information non chiffrée correspondant (SINC_{KOSO} de l'objet accessoire dé-identifié (KOS').

13. Equipement de communication (CSE) d'un site clinique apte à émettre et recevoir un objet d'information médicales dans ou d'un réseau de communication (RT), **caractérisé en ce qu'**il comprend un dispositif de ré-identification d'un objet d'informations médicales selon la revendication **10.**

14. Equipement de communication selon la revendication 13, **caractérisé en ce qu'**il comprend en outre un dispositif de dé-identification d'un objet d'informations médicales selon la revendication **12.**

15. Programme d'ordinateur (Pg1, Pg2) comportant des instructions pour la mise en œuvre du procédé selon l'une quelconque des revendications **1** à **9** et **11,** lorsque ledit programme est exécuté par un processeur.

## Patentansprüche

1. Verfahren zur erneuten Identifizierung eines Objekts mit medizinischen Informationen, bezeichnet als entidentifiziertes abgeleitetes Objekt (B'), empfangen von einer Ausrüstung (CSE) an einem Klinikstandort von einer Ausrüstung (NCSE) an einem Nicht-Klinikstandort über ein Kommunikationsnetz (RT), wobei das Objekt (B') ein Bild (I') umfasst, das von einem Ursprungsbild (I) abgeleitet ist, und mindestens einen ersten Verknüpfungs-Metadatenwert (MR_{A}) eines Objekts mit medizinischen Informationen, bezeichnet als entidentifiziertes Hauptobjekt (A'), das das Ursprungsbild (I) umfasst, wobei das Verfahren **dadurch gekennzeichnet ist, dass** es die folgenden Schritte umfasst:
- Erhalten (E2) des mindestens einen ersten Metadatenwerts (MR_{A'}) in einem nichtchiffrierten Informationsfeld (SINC_{B}) des entidentifizierten abgeleiteten Objekts (B');
- Erhalten (E3) mindestens eines zweiten Verknüpfungs-Metadatenwerts (MR_{A,ch}) eines Hauptobjekts (A), das das Ursprungsbild umfasst, wobei der mindestens eine zweite Metadatenwert in einem chiffrierten Informationsfeld (SIC_{C'}) enthalten ist, das einem zweiten entidentifizierten Objekt mit medizinischen Informationen (C', A', KOS') entspricht, wobei das zweite Objekt ferner den mindestens einen ersten Metadatenwert (MR_{A'}) in einem nichtchiffrierten Informationsfeld (SINC_{C'}) umfasst;
- Erzeugen (E4) eines abgeleiteten Objekts (B), das das abgeleitete Bild (I') umfasst, ausgehend von dem mindestens einen erhaltenen zweiten Metadatenwert (MR_{A,ch}).

2. Verfahren zur erneuten Identifizierung eines Objekts mit medizinischen Informationen nach Anspruch 1, **dadurch gekennzeichnet, dass** der Schritt des Erzeugens umfasst:
∘ ein Dechiffrieren (E41) des mindestens einen erhaltenen zweiten Metadatenwerts (MR_{A,ch});
∘ ein erneutes Kopieren (E42) des mindestens einen dechiffrierten zweiten Metadatenwerts (MR_{A}) in ein nichtchiffriertes Informationsfeld (SINC_{B}) des erneut identifizierten abgeleiteten Objekts (B), das dem des mindestens einen ersten Metadatenwerts entspricht; und
∘ ein erneutes Kopieren (E43) des abgeleiteten Bilds (I') in das abgeleitete Objekt (B).

3. Verfahren zur erneuten Identifizierung nach einem der Ansprüche **1** oder **2, dadurch gekennzeichnet, dass** der Schritt des Erhaltens (E3) von mindestens einem zweiten Metadatenwert einen Empfang (E31) des zweiten entidentifizierten Objekts mit medizinischen Informationen (C', A', KOS') von der Ausrüstung eines Nicht-Klinikstandorts (NCSE) und einen Vergleich (E32) von Metadatenwerten, die in nichtchiffrierten Informationsfeldern des zweiten entidentifizierten Objekts mit medizinischen Informationen enthalten sind, mit dem mindestens einen ersten Metadatenwert (MR_{A'}) umfasst, wobei der mindestens eine zweite Metadatenwert (MR_{A}, _{Ch}) erhalten wird, wenn eine Übereinstimmung zwischen dem mindestens einen ersten Metadatenwert und dem mindestens einen zweiten Metadatenwert gefunden ist.

4. Verfahren zur erneuten Identifizierung nach Anspruch **3**, **dadurch gekennzeichnet, dass** der Schritt des Erhaltens (E3') ferner das Senden (E30) einer Übertragungsanforderung des zweiten entidentifizierten Objekts mit medizinischen Informationen (C', A', KOS') an die Kommunikationsausrüstung (NCSE) umfasst, wobei die Anforderung den mindestens einen ersten Metadatenwert (MR_{A'}) umfasst.

5. Verfahren zur erneuten Identifizierung nach einem der Ansprüche **1** oder **2, dadurch gekennzeichnet, dass** der Schritt des Erhaltens (E3") das Senden (E31") einer Extraktionsanforderung des mindestens einen zweiten Metadatenwerts aus einem Feld chiffrierter Informationen des zweiten entidentifizierten Objekts mit medizinischen Informationen (A', KOS') umfasst, wobei die Anforderung den mindestens einen ersten Metadatenwert (MR_{A'}) umfasst.

6. Verfahren zur erneuten Identifizierung nach einem der vorangehenden Ansprüche, **dadurch gekennzeichnet, dass** das zweite entidentifizierte Objekt mit medizinischen Informationen (A') das Ursprungsbild (I) umfasst.

7. Verfahren zur erneuten Identifizierung nach einem der Ansprüche **1** bis **4, dadurch gekennzeichnet, dass** das zweite entidentifizierte Objekt mit medizinischen Informationen ein Objekt mit medizinischen Informationen (KOS') ist, bezeichnet als akzessorisches Objekt des entidentifizierten Hauptobjekts (A'), das mindestens einen Referenz-Metadatenwert zum Ursprungsbild (réfID_{A}) in einem Feld chiffrierter Informationen umfasst und kein Bild umfasst.

8. Verfahren zur erneuten Identifizierung nach einem der vorangehenden Ansprüche, **dadurch gekennzeichnet, dass**, wobei das entidentifizierte abgeleitete Objekt (B') ferner einen Metadatenwert umfasst, der eine Referenz (réfID_{A'}) zu einer Kennung (ID_{A'}) des Ursprungsbilds (I) im entidentifizierten Hauptobjekt (A') umfasst, das Verfahren das Erhalten (E2') einer Kennung des Ursprungsbilds (ID_{A}) in einem chiffrierten Informationsfeld (SINC_{C'}) umfasst, das dem zweiten Objekt entspricht, wobei der Schritt der erneuten Identifizierung des entidentifizierten abgeleiteten Objekts (B') die Dechiffrierung (E4') des Metadatenwerts und das erneute Kopieren (E6') des dechiffrierten Metadatenwerts in ein Referenzfeld (réfID_{A}) zum Ursprungsbild im erneut identifiizierten abgeleiteten Objekt (B) umfasst.

9. Verfahren zur erneuten Identifizierung nach einem der vorangehenden Ansprüche, **dadurch gekennzeichnet, dass**, wobei der Schritt des Erhaltens (E3) das Erhalten einer Sequenz zweiter chiffrierter Metadatenwerte (SIC_{C'}) umfasst, der Schritt des Dechiffrierens (E4) mindestens einen zweiten zusätzlichen Metadatenwert eines Felds der Sequenz dechiffriert, die keinem Feld eines ersten Metadatenwerts (MR_{B'}) entspricht, und der Schritt (E5) den zweiten zusätzlichen Metadatenwert erneut in das entsprechende nichtchiffrierte Feld des abgeleiteten Objekts (B) kopiert, wenn mindestens ein erster, aus dem empfangenen entidentifizierten abgeleiteten Objekt (B') extrahierter Metadatenwert einen Inklusionslink umfasst, der ihn mit dem zweiten zusätzlichen Metadatenwert verknüpft.

10. Vorrichtung (200) zur erneuten Identifizierung eines entidentifizierten Objekts mit medizinischen Informationen, bezeichnet als entidentifiziertes abgeleitetes Objekt (B'), empfangen von einer Kommunikationsausrüstung eines Klinikstandorts (CSE) von einer Ausrüstung (NCSE) eines Nicht-Klinikstandorts über ein Kommunikationsnetz (RT), wobei das Objekt ein abgeleitetes Bild (I') eines Ursprungsbilds (I) und mindestens einen ersten Verknüpfungs-Metadatenwert (MR_{A'}) eines entidentifizierten Objekts mit medizinischen Informationen, bezeichnet als entidentifiziertes Hauptobjekt (A'), das das Ursprungsbild umfasst (I), in einem nichtchiffrierten Informationsfeld umfasst, wobei die Vorrichtung **dadurch gekennzeichnet ist, dass** sie eine Rechenmaschine umfasst, die bestimmt oder ausgelegt ist, um:
- den mindestens einen ersten Metadatenwert (MR_{A}) im nichtchiffrierten Informationsfeld des entidentifizierten abgeleiteten Objekts (B') zu erhalten (OBT. MR_{A'});
- mindestens einen zweiten Verknüpfungs-Metadatenwert (MR_{A,ch}) eines Hauptobjekts (A), das das Ursprungsbild umfasst, zu erhalten (OBT. MR_{A'}, MR_{A,ch}), wobei der mindestens eine zweite Metadatenwert in einem chiffrierten Informationsfeld eines zweiten entidentifizierten Objekts mit medizinischen Informationen (C', A', KOS') enthalten ist, das den mindestens einen ersten Metadatenwert (MR_{A}) in einem nichtchiffrierten Informationsfeld (SINC_{C'}) umfasst;
- ein abgeleitetes Objekt (B) zu erzeugen (CREA. B), das das abgeleitete Bild (I') umfasst, ausgehend von dem mindestens einen erhaltenen zweiten Metadatenwert (MR_{A,ch}).

11. Verfahren zur Entidentifizierung eines Objekts mit medizinischen Informationen, bezeichnet als Hauptobjekt (A), durch eine Kommunikationsausrüstung eines Klinikstandorts (CSE) zwecks seiner Übertragung an eine Ausrüstung eines Nicht-Klinikstandorts (RSE) über ein Kommunikationsnetz, wobei das Objekt ein Ursprungsbild (I) und mindestens einen ersten Verknüpfungs-Metadatenwert (MR_{A}) in mindestens einem nichtchiffrierten Informationsfeld (SINC_{A}) umfasst, wobei das Verfahren die folgenden Schritte umfasst:
- Erzeugen eines entidentifizierten Hauptobjekts (A'), das das Ursprungsbild umfasst (I), wobei die Erzeugung umfasst:
∘ das Chiffrieren (T6) des mindestens einen ersten Metadatenwerts (MR_{A}) und das Speichern (T7) des mindestens einen ersten chiffrierten Metadatenwerts (MR_{A,ch}) in mindestens einem chiffrierten Informationsfeld (SIC_{A'}) des Objekts;
∘ das Erhalten (T8) von mindestens einem zweiten Metadatenwert (MR_{A}), der vom ersten Metadatenwert unterschiedlich ist, und sein Speichern in dem mindestens einen nichtchiffrierten Informationsfeld (SINC_{A'}) des entidentifizierten Hauptobjekts (A') anstelle des mindestens einen ersten Metadatenwerts (MR_{A});
wobei das Verfahren ferner die folgenden Schritte umfasst:
- Erzeugen eines Objekts mit medizinischen Informationen (KOS), bezeichnet als akzessorisches Objekt des Hauptobjekts (A), das den mindestens einen ersten Verknüpfungs-Metadatenwert (MR_{A}) des Hauptobjekts (A) in einem nichtchiffrierten Informationsfeld (SINC_{KOS}) umfasst, mindestens einen Referenz-Metadatenwert zum Ursprungsbild (réfID_{A}) in einem nichtchiffrierten Informationsfeld (SRNC_{KOS}) und kein Bild umfasst;
- Erzeugen eines entidentifizierten akzessorischen Objekts (KOS'), wobei das Erzeugen umfasst:
∘ das Chiffrieren des mindestens einen Referenz-Metadatenwerts (réfID_{A});
∘ das erneute Kopieren des mindestens einen chiffrierten Referenz-Metadatenwerts (refID_{A,Ch}) in ein entsprechendes chiffriertes Informationsfeld (SRC_{KOS'}) des entidentifizierten akzessorischen Objekts (KOS');
∘ das erneute Kopieren des mindestens einen chiffrierten ersten Metadatenwerts (MR_{A,ch}) des entidentifizierten Hauptobjekts (A') in mindestens ein chiffriertes Informationsfeld (SIC_{KOS'}) des entidentifizierten akzessorischen Objekts (KOS'); und
o das erneute Kopieren des mindestens einen zweiten Metadatenwerts (MR_{A'}) in das mindestens eine entsprechende nichtchiffrierte Informationsfeld (SINC_{KOS'}) des entidentifizierten akzessorischen Objekts (KOS').

12. Vorrichtung (200) zur Entidentifizierung eines Objekts mit medizinischen Informationen, bezeichnet als Hauptobjekt (A), durch eine Ausrüstung eines Klinikstandorts (CSE) zwecks seiner Übertragung an eine Ausrüstung eines Nicht-Klinikstandorts (RSE) über ein Kommunikationsnetz, wobei das Objekt ein Ursprungsbild (I) und mindestens einen ersten verknüpften Metadatenwert (MR_{A}) umfasst, wobei die Vorrichtung eine Rechenmaschine umfasst, die bestimmt oder ausgelegt ist, um:
- ein entidentifiziertes Hauptobjekt (A') zu erzeugen, umfassend:
∘ das Chiffrieren des mindestens einen ersten identifizierenden Metadatenwerts (MR_{A}) und das Speichern des mindestens einen ersten chiffrierten Metadatenwerts (MR_{A,ch}) in mindestens einem chiffrierten Informationsfeld (SIC_{A'}) des Objekts;
∘ das erneute Kopieren des mindestens einen zweiten Metadatenwerts (MR_{A'}) in das mindestens eine nichtchiffrierte Informationsfeld (SINCA') des entidentifizierten Hauptobjekts (A');
∘ das Speichern des mindestens einen Bilds (I) in dem entidentifizierten Hauptobjekt (A');
wobei die Vorrichtung ferner imstande ist:
- ein Objekt mit medizinischen Informationen (KOS), bezeichnet als akzessorisch zum Hauptobjekt (A), zu erzeugen, das den mindestens einen ersten identifizierenden Metadatenwert (MR_{A}) des Hauptobjekts (A) in einem nichtchiffrierten Informationsfeld (SINC_{KOS}) umfasst und kein Bild umfasst;
- ein entidentifiziertes akzessorisches Objekt (KOS') zu erzeugen, umfassend:
∘ Chiffrieren des mindestens einen Referenz-Metadatenwerts (réfID_{A});
∘ erneutes Kopieren des mindestens einen chiffrierten Referenz-Metadatenwerts in ein entsprechendes chiffriertes Informationsfeld (SRC_{KOS'}) des entidentifizierten akzessorischen Objekts (KOS');
∘ erneutes Kopieren des mindestens einen ersten chiffrierten Metadatenwerts (MR_{A,ch}) des entidentifizierten Hauptobjekts (A') in mindestens ein chiffriertes Informationsfeld (SIC_{KOS'}) des entidentifizierten akzessorischen Objekts (KOS'); und
o erneutes Kopieren des mindestens einen zweiten Metadatenwerts (MR_{A'}) in das mindestens eine entsprechende nichtchiffrierte Informationsfeld (SINC_{KOS'}) des entidentifizierten akzessorischen Objekts (KOS').

13. Kommunikationsausrüstung (CSE) eines Klinikstandorts, die imstande ist, ein Objekt mit medizinischen Informationen in oder von einem Kommunikationsnetz (RT) zu senden und zu empfangen, **dadurch gekennzeichnet, dass** sie eine Vorrichtung zur erneuten Identifizierung eines Objekts mit medizinischen Informationen nach Anspruch **10** umfasst.

14. Kommunikationsausrüstung nach Anspruch 13, **dadurch gekennzeichnet, dass** sie ferner eine Vorrichtung zur Entidentifizierung eines Objekts mit medizinischen Informationen nach Anspruch **12** umfasst.

15. Rechnerprogramm (Pg1, Pg2), das Befehle für die Durchführung des Verfahrens nach einem der Ansprüche **1** bis **9** und **11** umfasst, wenn das Programm von einem Prozessor ausgeführt wird.

## Claims

1. A method for re-identifying a medical information object, known as a de-identified derived object (B'), received by equipment (CSE) of a clinical site from equipment (NCSE) of a non-clinical site via a communication network (RT), said object (B') comprising an image (I') derived from an original image (I) and at least a first metadata (MR) relating to a medical information object, known as a de-identified derived object (B'), received by equipment (CSE) of a clinical site via a communication network (RT), said object (B') comprising an image (I') derived from an original image (I) and at least a first metadata (MR_{A'}) of a medical information object, said de-identified main object (A') comprising the original image (I), said method being **characterised in that** it comprises the following steps:
- obtaining (E2) said at least one first metadata (MR_{A'}) in an unencrypted information field (SINC_{B'}) of the de-identified derivative object (B');
- obtaining (E3) at least one second metadata (MR_{A,ch}) of attachment of a main object (A) comprising the original image, said at least one second metadata being contained in a corresponding encrypted information field (SIC_{C'}) of a second de-identified medical information object (C', A', KOS'), said second object further comprising said at least one first metadata (MR_{A'}) in an unencrypted information field (SINC_{C'});
- creating (E4) a derived object (B), comprising said derived image (I') from said at least one second metadata (MR_{A,ch}) obtained.

2. The method for re-identifying an object of medical information, according to claim 1, **characterised in that** the step for creating comprises:
∘ decrypting (E41) said at least one second metadata (MR_{A,ch}) obtained;
∘ copying (E42) said at least one second decrypted metadata (MR_{A}) into an unencrypted information field (SINC_{B}) of the re-identified derivative object (B) corresponding to that of said at least one first metadata; and
∘ copying (E43) the derived image (I') in the derived object (B).

3. The method of re-identification according to any of claims 1 or 2, **characterised in that** the step for obtaining (E3) at least a second metadata comprises a reception (E31) of the de-identified second medical information object (C', A', KOS') from the equipment of a non-clinical site (NCSE) and a comparison (E32) of metadata contained in unencrypted information fields of the de-identified second medical information object with said at least one first metadata (MR_{A'}), said at least one second metadata (MR_{A}, _{ch}) being obtained, when a match between said at least one first metadata and said at least one second metadata is found.

4. The method of re-identification according to claim 3, **characterised in that** the obtaining step (E3') further comprises sending (E30) a request for transmission of the de-identified second medical information object (C', A', KOS') to the communication equipment (NCSE), the request comprising said at least one first metadata (MR_{A'}).

5. The method of re-identification according to one of claims 1 or 2, **characterised in that** the obtaining step (E3") comprises transmitting (E31") a request for extracting said at least one second metadata from an encrypted information field of the de-identified second medical information object (A', KOS'), said request comprising said at least one first metadata (MR_{A'}).

6. The method of re-identification according to any of the preceding claims, **characterised in that** the de-identified second medical information object (A') comprises the original image (I).

7. The method of re-identification according to one of the claims to 1 to 4, **characterised in that** the second de-identified medical information object is a medical information object (KOS'), so-called accessory object of the main de-identified object (A'), which comprises at least one metadata reference to the original image (reflD_{A}) in an encrypted information field and does not comprise an image.

8. The method of re-identification according to any of the preceding claims, **characterised in that**, the de-identified derivative object (B') further comprising a metadata comprising a reference (reflD_{A'}) to an identifier (ID_{A'}) of the original image (I) in the de-identified main object (A'), the method comprises obtaining (E2') an identifier of the original image (ID_{A}) in a corresponding encrypted information field (SINC_{C}) of the second object, the step for re-identifying the de-identified derivative object (B') comprises decrypting (E4') said metadata and copying (E6') the decrypted metadata in a reference field (reflD_{A}) to the original image in the re-identified derivative object (B).

9. The method of re-identification according to one of the preceding claims, **characterised in that**, the obtaining step (E3) comprises obtaining a sequence of encrypted second metadata (SIC_{C'}), the decryption step (E4) decrypts at least one second so-called additional metadata of a field of said sequence which does not correspond to a field of a said first metadata (MR_{B'}) and the step (E5) recopies said second additional metadata into the corresponding unencrypted field of the derived object (B) when at least one first metadata extracted from the de-identified derived object (B') received comprises an inclusion link which attaches it to said second additional metadata.

10. A device (200) for re-identifying a de-identified medical information object, known as a de-identified derived object (B'), received by communication equipment of a clinical site (CSE) from equipment (NCSE) of a non-clinical site via a communication network (RT), said object comprising a derived image (I') of an original image (I) and at least a first metadata (MR) relating to a de-identified medical information object known as a de-identified derived object (B'), said object comprising a derived image (I') of an original image (I) and at least a first metadata (MR_{A'}) for attaching a de-identified medical information object called de-identified main object (A') comprising the original image (I) in an unencrypted information field, said device being **characterised in that** it comprises a computing machine dedicated to or configured for:
- obtaining (OBT. MR_{A'}) said at least one first metadata (MR_{A'}) in the unencrypted information field of the de-identified derivative object (B');
- obtaining (OBT. MR_{A'}, MR_{A,ch}) at least one second metadata (MR_{A,ch}) of attachment of a main object (A) comprising the original image, said at least one second metadata being contained in an encrypted information field of a de-identified second medical information object (C', A', KOS') comprising said at least one first metadata (MR_{A'}) in a non-encrypted information field (SINC_{C'});
- creating (CREA. B) a derived object (B) comprising said derived image (I') from said at least one second metadata (MR_{A,ch}) obtained.

11. A method for de-identifying a medical information object, referred to as the main object (A), by a communication equipment of a clinical site (CSE) with a view to its transmission to an equipment of a non-clinical site (RSE) via a communication network, said object comprising an original image (I) and at least a first attachment metadata (MR_{A}) in at least one non-encrypted information field (SINC_{A}), said method comprising the following steps:
- creating a de-identified main object (A') comprising the original image (I), said creating comprising:
∘ encrypting (T6) said at least one first metadata (MR_{A}) and storing (T7) said at least one first encrypted metadata (MR_{A,ch}) in at least one encrypted information field (SIC_{A'}) of said object;
∘ obtaining (T8) at least one second metadata (MR_{A'}) distinct from the first metadata and storing it in said at least one unencrypted information field (SINC_{A'}) of the de-identified main object (A'), instead of said at least one first metadata (MR_{A});
said method further comprising the following steps:
- creating a medical information object (KOS), known as an accessory object of the main object (A), comprising said at least one first metadata (MR_{A}) of attachment of the main object (A) in an unencrypted information field (SINC_{KOS}), at least one metadata of reference to the original image (reflD_{A}) in an unencrypted information field (SRNC_{KOS}) and not comprising an image;
- creating a de-identified accessory object (KOS'), said creation comprising:
∘ encrypting said at least one reference metadata (refID_{A});
∘ copying said at least one encrypted reference metadata (refID_{A,ch}) into a corresponding encrypted information field (SRC_{KOS'}) of the de-identified accessory object (KOS');
∘ copying said at least one first encrypted metadata (MR_{A,ch}) of the de-identified primary object (A') into at least one encrypted information field (SIC_{KOS'}) of the de-identified secondary object (KOS'); and
∘ copying said at least one second metadata (MR_{A'}) into the corresponding at least one unencrypted information field (SINC_{KOSO} of the de-identified accessory object (KOS').

12. A device (200) for de-identifying an object of medical information, called the main object (A), by an equipment of a clinical site (CSE) with a view to its transmission to an equipment of a non-clinical site (RSE) via a communication network, said object comprising an original image (I) and at least a first associated metadata (MR_{A}), said device comprising a computing machine dedicated to or configured to:
- create a de-identified main object (A') comprising:
∘ encrypting said at least one first identifying metadata (MR_{A}) and storing said at least one first encrypted metadata (MR_{A,ch}) in at least one information field (SIC_{A'}) of said object;
∘ copying said at least one second metadata (MR_{A'}) into said at least one unencrypted information field (SINCA') of the de-identified main object (A');
∘ storing said at least one image (I) in the de-identified main object (A');
said device is further adapted to:
- creating a medical information object (KOS), said accessory of the main object (A), comprising said at least one first identifying metadata (MR_{A}) of the main object (A) in a non-encrypted information field (SINC_{KOS}) and not comprising an image;
- creating a de-identified accessory object (KOS'), comprising:
∘ encrypting said at least one reference metadata (refID_{A});
∘ copying said at least one encrypted reference metadata into a corresponding encrypted information field (SRC_{KOS'}) of the de-identified ancillary object (KOS');
∘ copying said at least one first encrypted metadata (MR_{A,ch}) of the de-identified primary object (A') into at least one encrypted information field (SIC_{KOS'}) of the de-identified secondary object (KOS'); and
∘ copying said at least one second metadata (MR_{A'}) into said at least one corresponding unencrypted information field (SINC_{KOSO} of the de-identified ancillary object (KOS').

13. A communication equipment (CSE) of a clinical site capable of transmitting and receiving a medical information object in or from a communication network (RT), **characterised in that** it comprises a device for re-identifying a medical information object according to claim 10.

14. The communication equipment according to claim 13, **characterised in that** it further comprises a device for de-identifying an object of medical information according to claim 12.

15. A computer program (Pg1, Pg2) comprising instructions for implementing the method of any one of claims 1 to 9 and 11, when said program is executed by a processor.
